(19) Europäisches Patentamt
European Patent Office
Office européen des brevets

(11) EP 2 840 136 B1

(12) # EUROPEAN PATENT SPECIFICATION

(45) Date of publication and mention of the grant of the patent:
**08.02.2017 Bulletin 2017/06**

(21) Application number: **13764078.5**

(22) Date of filing: **22.03.2013**

(51) Int Cl.:
***C12Q 1/68*** *(2006.01)* ***C12N 15/09*** *(2006.01)*

(86) International application number:
**PCT/JP2013/058211**

(87) International publication number:
**WO 2013/141331 (26.09.2013 Gazette 2013/39)**

(54) **METHOD FOR DETECTING DNA HAVING MICROSATELLITE REGION**

VERFAHREN ZUM NACHWEIS VON DNA MIT MIKROSATELLITENREGION

PROCÉDÉ DE DÉTECTION D'UN ADN COMPORTANT UNE RÉGION MICROSATELLITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2012 JP 2012065232**

(43) Date of publication of application:
**25.02.2015 Bulletin 2015/09**

(73) Proprietors:
- **Wake Pure Chemical Industries, Ltd.**
  **Osaka-shi**
  **Osaka 540-8605 (JP)**
- **Kanagawa Prefectural Hospital Organization**
  **Kanagawa 231-0005 (JP)**

(72) Inventors:
- **MATSUKUMA, Shoichi**
  **Yokohama-shi**
  **Kanagawa 241-0815 (JP)**
- **ISHIKAWA, Tomokazu**
  **Amagasaki-shi**
  **Hyogo 661-0963 (JP)**
- **KUROSAWA, Tatsuo**
  **Amagasaki-shi**
  **Hyogo 661-0963 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(56) References cited:
**JP-A- H11 206 374** **JP-A- 2007 061 080**
**JP-A- 2007 190 016** **US-A- 5 753 439**

- **SHOICHI MATSUKUMA ET AL: "Differential detection ofmutations in codons 12 and 13 with a modified loop-hybrid (LH) mobility shift assay using an insert-type LH-generator", CLINICA CHIMICA ACTA, ELSEVIER BV, AMSTERDAM, NL, vol. 412, no. 19, 23 June 2011 (2011-06-23), pages 1874-1878, XP028246453, ISSN: 0009-8981, DOI: 10.1016/J.CCA.2011.06.030 [retrieved on 2011-06-30]**
- **SHOICHI MATSUKUMA ET AL: "Simple and precise detection of UGT1A1 polymorphisms with a modified loop-hybrid mobility shift assay using Cy5-labeled loop probes", CLINICA CHIMICA ACTA, vol. 412, no. 17-18, 1 August 2011 (2011-08-01), pages 1668-1672, XP055228290, AMSTERDAM, NL ISSN: 0009-8981, DOI: 10.1016/j.cca.2011.05.021**
- **SHOICHI MATSUKUMA: 'Loop Hetero Duplex DNA no PAGE deno Kyodo Henka o Riyo shita Idenshi Hen'i Kenshutsu' SEIBUTSU BUTSURI KAGAKU vol. 52, no. 3, 15 September 2008, page 75, XP008174377**
- **MATSUKUMA, S. ET AL.: 'Rapid and simple detection of hot spot point mutations of epidermal growth factor receptor, BRAF, and NRAS in cancers using the loop-hybrid mobility shift assay.' J. MOL. DIAGN. vol. 8, no. 4, September 2006, pages 504 - 512, XP002718858**
- **TOSHIHARU MAKI: 'Apprication 2-5 Microsatellite Takei Kaiseki, Saibo Kogaku Bessatsu Tips Seriese revised edition PCR Tips' KANOSEI O HIROGERU KOTSU TO HINT 10 December 1999, pages 130 - 135, XP008174664**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **C F SPINK ET AL: "Discrimination of suballeles present at the TNFd microsatellite locus using induced heteroduplex analysis", GENES AND IMMUNITY, vol. 5, no. 1, 1 January 2004 (2004-01-01) , pages 76-79, XP055288373, GB ISSN: 1466-4879, DOI: 10.1038/sj.gene.6364039**

## Description

[TECHNICAL FIELD]

The present invention relates to a method for detecting DNA having a microsatellite region using a loop hybrid method, and a hybrid obtained by a loop hybrid method.

[BACKGROUND ART]

Repeated sequences in which 1 to 6 nucleotides make one unit and they are repeatedly formed, are called as the microsatellite regions, and exist in many mammal animals, for example, human genome sequence etc. Thus, it is known that there exist 3000 or more sites in human genome. It is known that this microsatellite region is useful as a marker of the various genetic analysis because the number of iteration (number of cycles) thereof exhibits different polymorphism. Also, when the microsatellite region exists in the protein code region of gene, it is known that difference in the number of cycles thereof causes the disease, therefore, various studies on the microsatellite region have been carried out in order to investigate a correlation between gene and disease.

A method for analyzing polymorphism of DNA having such microsatellite region is usually carried out by PCR amplification, and then followed by separation analysis of fragment thereof by modified polyacrylamide gel electrophoresis, or capillary electrophoresis. However, when analyzing the polymorphism of microsatellite DNA, electrophoresis using 30 cm capillary, or electrophoresis using 40 cm length of modified polyacrylamide gel was required. Therefore, there was such a problem that it becomes expensive and needed specialized technique.

On the other hand, as a method for detecting a mutant gene, a loop hybrid method (LH method) is known that a single-stranded oligo-DNA is added into the reaction solution after PCR reaction of DNA fragment to hybridize with the objective DNA fragment, and to separate by electrophoresis on the basis of the structural difference of the obtained hybrid, and to discriminate the mutant gene (refer to Patent Literature 1). In addition, by applying said LH method, detection of the mutant gene of UGT1A1 having the microsatellite region has been carried out (refer to Non-patent Literature 1) as well.

[Citation List]

[Patent Literature]

[Patent Literature 1] JP-2007-61080 A;

[Non-Patent Literature]

[Non-Patent Literature 1] Clinica Chimica Acta, 412, 1688-1672;

[DISCLOSURE OF INVENTION]

[TECHNICAL PROBLEM]

The present inventors have advanced the research on the method described in non-patent literature 1 (conventional LH method) because there is no problem that the expensive instrument or the specialized technique is required. However, when said conventional LH method is used, because non-specific reaction products other than the objective hybrid are much produced, the present inventors have found that the problems that it is difficult to specify the objective substance have occurred. That is, the conventional method has aimed only to separate the wild type DNA from the mutant DNA, and therefore, the problem has not occurred when these migrating band or migration peak is apart from that of the non-specific reaction product. However, when polymorphism is separated by the difference of the iteration number in the microsatellite region, it was found that the non-specific reaction product can hinder to detect the objective substance. From this situation, the purpose of the present invention is to provide the method that can accurately detect DNA having microsatellite region by using LH method forming the hybrid having the specific loop structure, without causing the above problem of non-specific reaction product.

[SOLUTION TO PROBLEM]

That is, as a result of intensive research on the detection method of DNA having the microsatellite region using LH method, the present inventors have found that non-specific reaction product can hardly be produced on hybridizing the probe with the objective DNA, by synthesizing a probe so that all of the microsatellite regions enters into the loop structure, and thus, have completed the present invention. That is, the present invention relates to: "a method for detecting DNA having a microsatellite region by

(1) contacting a probe, which does not have a nucleotide sequence complementary to said microsatellite region and hybridizes with both sides of nucleotide sequences of said microsatellite region, with DNA having the microsatellite region, to form a hybrid of the above-mentioned DNA and the above-mentioned probe, which has a loop structure including microsatellite region,
(2) separating the obtained hybrid based on the difference of molecular weight , molecular structure and/or charge,
(3) detecting said hybrid" and wherein "a hybrid of DNA and a probe, having a loop structure including the microsatellite region, is made by contacting DNA having microsatellite region with the probe which does not have the nucleotide sequence complementary to said microsatellite region, and hybridizes with both sides of nucleotide sequence of said microsatellite region", and wherein the number of repetitions of a unit as a standard of iteration in the microsatellite region is 5 to 100.

[EFFECT OF THE INVENTION]

According to the present invention, DNA having the microsatellite region can be detected simply and accurately.

In addition, even when DNA having the microsatellite region having the different number of cycles (iteration number) is used, this method can separate it depending on the length of number of cycles (iteration number), and hardly produces the non-specific reaction product which has produced much in case of employing the conventional LH method. Therefore, it is possible to detect the objective DNA without detection error and with higher accuracy.

Particularly, it was found that by designing the probe so that all of the microsatellite regions enter into the loop structure, non-specific reaction product is hardly produced, and the mutation polymorphism having the different iteration number can be detected according to the difference of the iteration number. This is quite unexpected even by the present inventors.

That is, the detection of the presence or absence of one nucleotide substitution was a main object in the conventional method, and therefore, the probe was designed so that the substituted nucleotide exists in the root portion (starting portion) of the loop structure. Thus, if there is a mutation in the microsatellite region, the probe was designed so that the microsatellite region exists in the root portion of the loop structure, that is, a portion of the microsatellite region binds to the probe and to partially form a loop structure. Therefore, it is unexpected that by designing the probe so that all of the microsatellite regions enter into the loop structure, non-specific reaction product is hardly produced, and at the same time the mutation polymorphism having the different iteration number can be detected depending on the difference of the iteration number.

[BRIEF DESCRIPTION OF DRAWINGS]

Fig. 1 shows the separation result by electrophoresis of LH reaction product obtained according to the present invention method using the poly-T strand length polymorphism of 9 kinds of TOMM 40 gene.
Fig. 2 shows the separation result by electrophoresis of LH reaction product obtained according to the conventional LH method using the poly-T strand length polymorphism of 9 kinds of TOMM 40 gene.
Fig. 3 shows the separation result (3-a) by electrophoresis of LH reaction product obtained according to the present invention method , and the separation result (3-b and 3-c) by electrophoresis of LH reaction product obtained according to the conventional LH method, using the strand length poly-T polymorphism of 3 kinds of TOMM 40 gene.
Fig. 4 shows the separation result by electrophoresis of the LH reaction product obtained according to the present invention method using CAG repeat polymorphism in 5 kinds of androgen receptor (AG) gene sequences.
Fig. 5 shows the separation result by electrophoresis of the LH reaction product obtained according to the conventional LH method using CAG repeat polymorphism in 8 kinds of androgen receptor (AG) gene sequences.

[DESCRIPTION OF EMBODIMENTS]

<u>[DNA having the microsatellite region relevant to the present invention]</u>

The microsatellite region relevant to the present invention shows a region consisting of the repetitive nucleotide sequence wherein normally 1 to 6 nucleotides, preferably 1 to 3 nucleotides make one unit, and it is repetitively formed. Repetition number (iteration number) of the sequence of one unit (hereinafter, abbreviated as a unit sequence) as a standard of iteration in the repetitive nucleotide sequence, is usually 5 to 100, preferably 5 to 50. In addition, if the unit sequence of the repetitive nucleotide sequence is 1 nucleotide, the number of cycles thereof is at least 3 or more. This is why the loop structure cannot be formed unless at least 3 or more nucleotides exist because the loop structure in the hybrid of the present invention may be composed only of the microsatellite region. Nucleotide number in said microsatellite region is usually 3 to 600 nucleotides, preferably 5 to 600 nucleotides, more preferably 5 to 300 nucleotides. A specific sequence in the above-mentioned microsatellite, in the case when the unit sequence of the repetitive nucleotide sequence is one nucleotide, includes Poly T or Poly A or the like, in case of 2 nucleotides, includes the one consisting of CA repeat, or the like, and in case of 3 nucleotides, includes the one consisting of CAG, CTG, and CGG repeats, or the like.

As for DNA having the microsatellite region relevant to the present invention (hereinafter, it may simply be abbreviated as DNA relevant to the present invention), so long as it is the one having the above microsatellite region, and it is the single-stranded DNA wherein the nucleotide sequences of usually 5 to 1000 nucleotides, preferably 5 to 500 nucleotides, more preferably 5 to 100 nucleotides, anteroposterior of the microsatellite region thereof, are the known one, any DNA may be all right. Among them, in the microsatellite region, DNA (polymorphism DNA) in which iteration number of unit sequence of the microsatellite region is different is more preferable. A specific DNA includes a genomic DNA fragment isolated from an organism such as animal, microorganism, bacteria, or plant, a DNA fragment isolatable from virus, and a cDNA fragment synthesized from mRNA as a template, or the like, among them, cancer gene derived from human cell is preferably included. In addition, strand length of the above DNA (strand length to be detected) is usually 13 to 2000 nucleotides, preferable 13 to 1000 nucleotides, more preferably 13 to 200 nucleotides. It should be noted that, as described later, the probe of the present invention is produced so as to set the specific microsatellite region as detection target, thus, the DNA relevant to the present invention may have one or more microsatellite region (the second microsatellite region) other than the microsatellite region which is a detection target, however, as for the second microsatellite region, the one in which unit sequence thereof is different from the unit sequence in the microsatellite region, which is a detection target, is preferable.

As for the above DNA, the one which is purified as much as possible, and unwanted components other than DNA fragment have been removed, is preferable. Specifically, for example, the one which is purified according to the conventional method, such as Boom method using silica carrier (Boom et al. J. Clin. Microbiol. 28:495-503 (1990)), or a method using solution of sodium iodide (Proc. Natl. Acad. Sci. USA 76-2, p 615-619 (1979)), is preferable. In addition, the one in which the objective DNA is amplified by the polymerase chain reaction (PCR reaction) known per se, for example, by the method described in Nucleic Acids Research, 1991, Vol. 19, 3749; Bio Techniques, 1994, Vol. 16, 1134-1137, may be used.

If the DNA relevant to the present invention is forming double-strand, the DNA relevant to the present invention may be obtained by converting the double-stranded DNA into single-strand by heating treatment (90 to 100 °C) or alkali treatment or the like (treatment by sodium hydroxide or the like) usually performing in this field.

<u>[Probe relevant to the present invention]</u>

Probe relevant to the present invention is the one which forms the hybrid having loop structure by hybridizing with the DNA relevant to the present invention, and does not have the nucleotide sequence complementary to the microsatellite which is a detection target. That is, it is the nucleotide sequence which does not bind to the microsatellite region which is a detection target, and bind to both sides of nucleotide sequence of the microsatellite region. For example, there is included the complementary strand of the sequence excluded the microsatellite region from the DNA relevant to the present invention or the complementary strand of the sequence excluded the microsatellite region and 1 to 10 nucleotides in the upstream and downstream thereof from DNA having the microsatellite region relevant to the present invention, or the like. As for the probe relevant to the present invention, more specifically, it is included the sequence which is made by binding the complementary strand of nucleotide sequence having usually 5 to 1000 nucleotides, preferably 5 to 200 nucleotides, more preferably 5 to 100 nucleotides which from 3'-terminal of the microsatellite region toward the 3'-terminal direction of DNA having the microsatellite region, starts from a nucleotide next to 1 to 11 nucleotides, and the complementary strand of nucleotide sequence having usually 5 to 1000 nucleotides, preferably 5 to 200 nucleotides, more preferably 5 to 100 nucleotides which from 5'-terminal of the microsatellite region toward 5'-terminal direction of DNA having the microsatellite region, starts from a nucleotide next to 1 to 11 nucleotides so that the complementary nucleotide of a nucleotide next to 1 to 11 nucleotides from 3'-terminal of the microsatellite region; and the complementary nucleotide of a nucleotide next to 1 to 11 nucleotides from 5'-terminal of the microsatellite region are bound. Such a probe forms hybrid having the loop structure when hybridizing with the DNA relevant to the present invention. In this case, the loop structure relevant to the present invention is composed only of the microsatellite region, or is composed of the microsatellite region and nucleotides of 1 to 10 nucleotides at both sides or one side thereof.

The probe relevant to the present invention is that the sequence which is made by binding the complementary strand of nucleotide sequence of usually 5 to 1000 nucleotides, preferably 5 to 200 nucleotides, more preferably 5 to 100 nucleotides which, from 3'-terminal of the microsatellite region toward 3'-terminal direction of DNA having the microsatellite region, starts from a nucleotide next to 1 nucleotide, and the complementary strand of nucleotide sequence having usually 5 to 1000 nucleotides, preferably 5 to 200 nucleotides, more preferably 5 to 100 nucleotides which, from 5'-terminal of the microsatellite region toward 5'-terminal direction of DNA having the microsatellite region, starts from a nucleotide next to 1 nucleotide so that the complementary nucleotide of a nucleotide next to 1 nucleotide from 3'-terminal in the microsatellite region, and complementary nucleotide of nucleotide next to 1 nucleotide from 5'-terminal in the microsatellite region are bound, is preferable. In this case, the loop structure relevant to the present invention is composed only of the microsatellite region.

It should be noted that, as for the hybrid relevant to the present invention, it is preferable to carry out the end blunting treatment as described below in the detection method of the present invention when the hybrid has protruding end. Therefore, it is not necessary for the probe to bind all the nucleotide sequence excluding the microsatellite region, or the microsatellite region and the nucleotide of 1 to 20 nucleotides next to it, from the nucleotide sequence of DNA relevant to the present invention

Strand length of the probe relevant to the present invention is usually 10 to 2000 nucleotides, preferable 10 to 1000 nucleotides, more preferably 10 to 200 nucleotides.

By hybridizing with DNA relevant to the above present invention using the above probe, the hybrid which has the loop structure composed of the microsatellite region, or the microsatellite region and nucleotide sequence of 1 to 10 nucleotides of one side or both sides next to 3'-terminal or/and 5'-terminal thereof, on DNA relevant to the present invention, can be formed.

In addition, when the DNA relevant to the present invention has the second microsatellite region, on forming the hybrid, the probe relevant to the present invention may be either the one forming double strand with said second microsatellite region or the one making said second microsatellite region to the loop structure. When any of the unit sequence in said second microsatellite region is the same as that in microsatellite region which is a detection target, the probe relevant to the present invention is preferably the one making said second microsatellite region to the loop structure. This is because it enables to avoid the possibility that such linkage error that the probe binds to another repetitive sequence other than the set repetitive sequence may be occurred, and a plurality of non-specific reaction products may be produced. The loop structure formed by said second microsatellite region is preferably the one which includes all of the second microsatellite regions, however, if the complementary strand (complementary nucleotide) of the same unit sequence as the microsatellite region which is a detection target, in the probe does not exist, it may be the one which contains a part of the microsatellite region. When such a probe is used, the obtained hybrid consequently forms a plurality of loop structures.

<u>[Hybrid of the present invention]</u>

The hybrid of the present invention is the one obtained by binding (hybridizing) the probe relevant to the present invention to the DNA relevant to the present invention, as described above, and it is the one which forms the loop structure on the DNA relevant to the present invention, and said loop structure includes all of the microsatellite regions. That is, said hybrid is composed of 2 sites of double-stranded nucleotide parts and the loop structure of single-stranded nucleotide parts which are sandwiched with them (existing between them). It should be noted that, said hybrid may have the protruding end, the one having no protruding end is preferable because it is possible to effect the separation. In the site which is sandwiched between 2 sites of the double-stranded nucleotide parts, nucleotide chain at opposite side of nucleotide chain having the loop structure may have single-stranded nucleotide having 1 to 2 nucleotides. In addition, said hybrid may be the one which further forms another loop structure, and has the loop structures on both sides of the hybrid. Furthermore, in the case that the DNA relevant to the present invention has the second microsatellite region, among them, at least one of unit sequences becomes the same as that of the microsatellite region, which is a detection target, it is preferable to further form the loop structure formed by all or part of the second satellite region on the DNA relevant to the present invention.

The above-mentioned loop structure is composed of single-stranded nucleotide that does not hybridize with probe (not forming base pair) formed on genome DNA, and is the one forming loop secondary structure (steric structure) in the hybrid. Said loop structure is composed of usually 3 to 600 nucleotides, preferably 5 to 600 nucleotides, more preferably 5 to 300 nucleotides.

The double-stranded nucleotide parts at 2 sites described above are independently each other composed of usually 5 to 1000 base pairs, preferably 5 to 500 base pairs, more preferably 5 to 100 base pairs. It should be noted that, said double-stranded nucleotides may have the protruding end according to the kind of the probe or specification of LH method described later.

As described above, the hybrid of the present invention is the one including all of the microsatellite regions, therefore, in the case that the microsatellite region detects different DNA polymorphism, if the hybrid is formed by using the same probe, shape, size, charge or the like of the loop structure formed are different. Thus, by separating the hybrid including DNA, in which the microsatellite region is different, by the difference of molecular weight, the difference of molecular structure or/and the difference of charge, these can be accurately discriminated. While the hybrid in the conventional LH method has also formed the loop structure, all of the microsatellite regions have not been included in the loop structure, accordingly, the hybrid (no-specific reaction product) other than the objective hybrid has been produced, and these non-specific reaction products have inhibited the detection of the objective hybrid. Reason why this non-specific reaction product is produced, is not clear, however, it is considered that the complementary sequences of said probe bind to the repetitive sequences present in the multiple microsatellite regions because the probe includes the complementary sequence in a part of the microsatellite region so as to bind to a part of the microsatellite region when forming the hybrid, and then various hybrids (non-specific reaction product) having the different loop structure are formed. That is, the complementary sequence of the repetitive sequence in the probe binds to another repetitive sequence other than the set repetitive sequence, therefore, it is speculated that various non-specific reaction products are produced.

Schematic diagram of the hybrid of the present invention, when hybrid is formed by using the probe of the present invention, is shown below. That is, in said case, the probe is designed by binding the complementary nucleotide sequence (A and B) to both sides nucleotide sequence (A' and B') of satellite region of the DNA relevant to the present invention. When the DNA relevant to the present invention is hybridized by using said probe, the microsatellite region becomes the loop structure, and the hybrid having the loop structure including all of the microsatellite regions is formed.

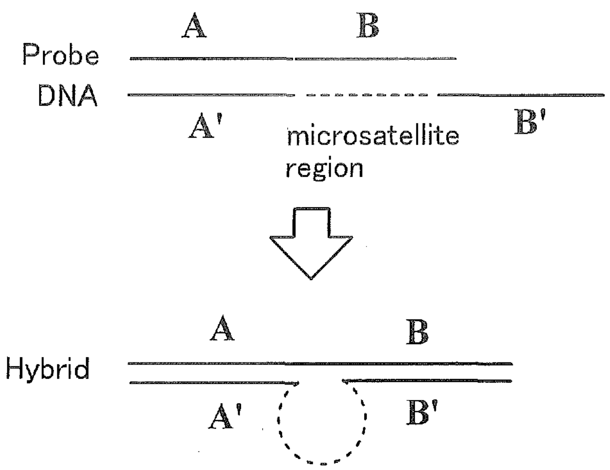


[Method for detecting DNA relevant to the present invention (Method of the present invention)]

A method for detecting the DNA relevant to the present invention is carried out by (1) forming the hybrid of the present invention by contacting the probe relevant to the present invention with the DNA relevant to the present invention, (2) separating the obtained hybrid and (3) detecting said hybrid to detect DNA having the microsatellite region.

As for a method forming for the hybrid in the above-mentioned (1) (hereinafter, said reaction will be abbreviated as LH reaction), the hybrid may be formed by making the DNA relevant to the present invention contact with the probe relevant to the present invention, and subjecting them to DNA molecule association reaction to form the hybrid. It should be noted that, in case that the DNA relevant to the present invention forms double-strand, after double-stranded DNA is converted to single-strand by heat-treatment (90-100°C) or alkali treatment performed usually in this field to form the DNA relevant to the present invention, it may be subjected to LH reaction by contacting said DNA with the probe relevant to the present invention. In addition, in case that the hybrid obtained in said LH reaction has a protruding end, said protruding end is preferably subjected to end blunting treatment. Thereby, it can be avoided to produce the unexpected non-specific reaction products due to existence of single-strand because there exists no single-strand at terminal of hybrid. As for said end blunting treatment, there is exemplified DNA extension reaction by using enzyme having polymerase activity, or decomposition reaction of terminal single-stranded DNA by using enzyme having exonuclease activity, or the like. Among them, the hybrid of the present invention is more stable when the hybrid has a long double-stranded nucleotide part, the DNA extension reaction is preferable.

The above-mentioned DNA association molecule reaction is carried out by adding the probe relevant to the present invention into water or a buffer solution containing the DNA relevant to the present invention to adjust the concentration in solution to 20 nM to 2 μM, preferably 100 nM to 500 nM, and by reacting usually at 30 to 55 °C, for 1 to 600 seconds, preferably 1 to 30 seconds. As for the water containing the DNA relevant to the present invention, deionized sterilized water is preferable, the buffer solution is not particularly limited so long as it is the known per se the buffer solution used in this field, for example, it includes Tris buffer solution, phosphate buffer solution, veronal buffer solution, borate buffer solution, Good's buffer solution, or the like, and pH thereof is not particularly limited, and it is usually in range of 5 to 9.

The above-mentioned end blunting reaction may be carried out according to the known per se blunting reaction, for example, 1 ng to 1 μg of hybrid is added to 20 to 40 μL of water or a buffer solution, further, usually 0.01 to 50 nmol, preferably 0.1 to 20 nmol of 4 kinds of deoxyribonucleotide triphosphate (dNTPs) respectively, and 1 to 5U of enzyme having exonulease activity are added, then, solution is reacted at usually 30 to 70 °C, preferably 35 to 60 °C, for usually 10 to 120 minutes, preferably 30 to 60 minutes. The water or the buffer solution in said reaction includes the same one described in paragraph of the above-mentioned molecular association reaction. An enzyme having the above-mentioned exonuclease activity includes, for example, T4 DNA polymerase, KOD DNA polymerase, pfu DNA polymerase, or the like.

The above-mentioned DNA extension reaction may be carried out by adding 1 ng to 1 μg of the hybrid obtained by the molecular association reaction to 20 to 40μL of water or a buffer solution, further, adding 1 to 5 U of enzyme having polymerase activity, if necessary, usually 0.01 to 50 nmol, preferably 0.1 to 20 nmol of 4 kinds of deoxyribonucleotide triphosphate (dNTPs) respectively together with, and reacting at usually 30 to 80 °C, preferably 65 to 75 °C, for usually 10 sec to 10 minutes, preferably 1 to 4 minutes. The water or the buffer solution in said reaction includes the same one described in paragraph of the above-mentioned molecular association reaction. In addition, the enzyme having polymerase activity includes, for example, T4 DNA polymerase, KOD DNA polymerase, pfu DNA polymerase, Taq DNA polymerase, Klenow fragment, or the like. Among them, heat resistant DNA polymerase such as Taq DNA polymerase, KOD DNA polymerase is preferable.

As for the DNA relevant to the present invention in the above (1) reaction, the one amplified according to the known PCR reaction may be used. In this case, PCR reaction product is double-stranded DNA, and therefore, after single-strand formation of the above-mentioned double stranded DNA, it may be subjected to LH reaction. In addition, in case of using PCR reaction solution including PCR reaction product, and being subjected to LH reaction, and to DNA extension reaction as it is, reaction can be carried out without adding dNTPs or enzyme having polymerase activity, therefore, using said method is preferable.

LH reaction relevant to the present invention is preferable to perform as cycle reaction by adding the single-strand formation treatment of double-stranded DNA by heating before the molecular association reaction. Specifically, for example, the probe relevant to the present invention is added to the water or the buffer solution having the DNA relevant to the present invention to adjust the concentration in solution to 20 nM to 2 μM, preferably 100 nM to 500 nM, and then, for example, by setting reaction condition of carrying out at 90 to 100 °C for 2 to 4 minutes (thermal denaturation), at 30 to 55 °C for 1 to 30 secconds (the molecular association reaction) as 1 cycle, hybrid may be formed by performing the reaction for 1 to 4 cycles. In addition, further, the cycle reaction may be performed by adding extension reaction, in this case, reaction by setting condition of carrying out at 90 to 100 °C for 2 to 4 minutes (thermal denaturation), at 30 to 55 °C for 1 to 30 seconds (DNA molecule association reaction), and at 65 to 75 °C for 1 to 4 minutes (DNA extension reaction) as 1 cycle, hybrid may be formed by performing for 1 to 4 cycles. Further, when carrying out the extension reaction, since DNA molecule association reaction can procees even under the condition of DNA extension reaction, by setting the reaction condition of carrying out at 90 to 100 °C for 2 to 4 minutes (thermal denaturation), and at 65 to 75 °C for 1 to 4 minutes (DNA molecular association, DNA extension reaction), is specified as 1 cycle, by performing the reaction for 1 to 4 cycles, LH reaction may be carried out.

The Cycle reaction of thermal denaturation, the molecular association reaction and DNA extension reaction, specifically, is carried out as bellow. That is, first of all, for example, into 20 to 40 μL of buffer solution such as 10 to 50 mM Tris buffer solution (pH: 8.4 to 9.0) in which 100 ng of DNA having the microsatellite region is dissolved, the probe relevant to the present invention is added to adjust the concentration in solution to 20 nM to 2 μM, preferably 100 nM to 500 nM, more preferably 100 nM to 200 nM. Then, for example, the reaction condition is set at 90 to 100 °C for 2 to 4 min (thermal denaturation), at 30 to 55 °C for 1 to 30 sec (DNA molecule association reaction), and at 65 to 75 °C for 1 to 4 min (DNA extension reaction) as 1 cycle, and hybrid may be formed by performing the reaction for 1 to 4 cycle.

As described above, in case of amplifying the DNA relevant to the present invention by PCR reaction, followed by being subjected to LH reaction, step (1) including LH reaction is specifically carried out as described below. That is, for example, 1 to 100 pg of DNA as template is dissolved in buffer solution such as 20 to 40 μL of Tris buffer solution, and 2 kinds of primers (Forward, Reverse) for amplifying the objective region are added to adjust to usually 1 to 100 pmol, preferably 1 to 50 pmol, respectively, and 4 kinds of deoxyribonuclease triphosphate (dNTPs) are added to adjust to usually 0.01 to 50 nmol, preferably 0.1 to 20 nmol, respectively, and 1 to 5U of heat resistant polymerase such as Taq DNA polymerase, KOD DNA polymerase is made coexist, and for example, reaction condition of carrying out (1) at 93 to 98 °C for 10 seconds to 10 minutes, (2) at 50 to 60 °C for 10 seconds to 3 minutes, then at 65 to 75 °C for 1 to 5 minutes is specified as 1 cycle, and reaction is carried out by performing 30 to 40 cycles. Then, into the obtained reaction solution, the probe relevant to the present invention is added by 0.1 to 10 times amount of DNA as template, for example, usually 0.1 to 500 pmol, preferably 0.1 to 50 pmol, and LH reaction is carried out as described above,

In addition, in case of amplifying a single-stranded DNA by PCR reaction, LH reaction can be carried out simultaneously with said PCR reaction. In this case, as for the probe relevant to the present invention, the one modified at 3'-terminal and 5'-terminal with phosphate group or the like is used, and the above-mentioned PCR reaction may be carried out under the presence of said probe. Specifically, for example, 1 to 100 pg of DNA as template is dissolved in buffer solution such as 20 to 40 μL of 10 to 50 mM Tris buffer solution (pH 8.4 to 9.0), and usually 1 to 100 pmol, preferably 1 to 50 pmol of 2 kinds of primers (Forward, Reverse), respectively, and usually 0.01 to 50 nmol, preferably 0.1 to 20 nmol of 4 kinds of deoxyribonucleotide triphosphate (dNTPs), respectively, and usually 0.1 to 500 pmol, preferably 0.1 to 50 pmol of the probe relevant to the present invention modified at 3'-terminal and 5'-terminal are added, and for example, 1 to 5U of heat resistant polymerase is made coexist, for example, reactions (1) at 93 to 98 °C for 10 sec to 10 min, (2) at 50 to 60 °C for 10 seconds to 3 minutes, then (3) at 65 to 75 °C for 1 to 5 minutes is set as 1 cycle, and reaction is carried out by performing 30 to 40 cycles.

A method for separating the above-mentioned hybrid is not particularly limited so long as it is the method for separating DNA to be used usually in this field, particularly, the method by which double-stranded DNA may be separated, the method for separating based on the difference of molecular weight, molecular structure or/and charge is preferable. That is, in case that the hybrid of the present invention is formed by using a plurality of DNA polymorphisms having different microsatellite region, the obtained plural hybrids are the different ones in size of loop structure or/and charge. Further, in case when forming the second structure which forms partially complementary base pair in the loop structure, there may be the one which the second structure thereof becomes different. Therefore, it is possible to accurately separate by separating the above-mentioned plural hybrids based on the difference of molecular weight, molecular structure or/and charge, or the like.

It should be noted that, separation described herein, specifically, means separation of 2 or more the hybrid of DNA polymorphisms having the different microsatellite region, and, the case which one kind of the hybrid of the present invention is subjected to the above separation method may also be included.

As for a method for separating based on the difference of the above-mentioned molecular weight, molecular structure or/and charge, specifically, for example, there are included a high-pressure liquid chromatography (HPLC method), an electrophoresis method, a separation method using filter or the like. Among them, the electrophoresis method is more preferable.

In case of employing HPLC method, for example, it may be carried out according to the method described in Anal. Chem. 65, 5,613-616(1993), WO 03/014398, WO 03/031580, US 5585236, US 5772889, US 5972222 or the like (Ion-pair reverse-phase high pressure liquid chromatography, Matched ion polynucleotide chromatography or the like. In addition, in case that separation method using filter is employed, for example, it may be carried out according to the method described in Panasonic Technical Journal Vol. 57 No. 3 Oct. 2011, or JP 2009-125009 A or the like.

As for the electrophoresis method, more specifically, for example, there are included an electrophoresis method such as an isoelectric point electrophoresis method, a SDS-polyacrylamide electrophoresis method, an agarose gel electrophoresis method, an acrylamide electrophoresis method, a capillary electrophoresis method, a capillary-tip electrophoresis method, and an dielectrophoresis method. Among them, from such reasons that cooling efficiency is excellent, high voltage can be loaded, and separation is efficiently carried out, the capillary electrophoresis method, the capillary-tip electrophoresis method are preferable, and capillary-tip electrophoresis method suitable for micro-sample analysis is particularly preferable. It should be noted that, conditions of these separation methods may be carried out according to the known per se method, for example, capillary-tip electrophoresis method may be carried out according to the method described in WO 2007/027495 or the like. It should be noted that, length of capillary electrophoresis or polyacrylamide gel is sufficient for usually 1 to 20 cm, preferably 1 to 10 cm.

The method for detecting the hybrid in the above-mentioned (3) may be detected according to the method for detecting the hybrid in the above-mentioned (2). For example, in case of employing HPLC method, the hybrid relevant to the present invention may be detected based on mobility or elution time. For example, in case of employing the method using filter, the hybrid relevant to the present invention may be detected based on transit time of filter. In case of employing the electrophoresis, the hybrid relevant to the present invention may be detected based on migration time or migration distance (travelling time or travelling distance). More specifically, for example, 2 or more DNA polymorphisms having different microsatellite region are separated by electrophoresis, and the hybrid relevant to the present invention may be detected based on migration time or migration distance.

As for the detection method in the above-mentioned (3), any method can be used as long as it is the known per se method, and it may be detected by instrument such as differential refraction detector, fluorescence detector, UV detector. Among them, detection by UV detector, fluorescence detector are preferable, detection by fluorescence detector is more preferable.

In case of carrying out the fluorescence detection, it is required to performing the fluorescence labelling to the detection targeting substance, and it may be carried out according to the method conventionally used in this field. Specifically, for example, it is included the method for detecting fluorescence by using the probe fluorescence labeled in advance, or the like. In case of separating the hybrid by electrophoresis, the method labelling DNA by intercalator or the like after separation may be carried out.

The method for fluorescence labeling the probe includes, for example, labelling by using cyanine dye. Cyanine dye described herein is a compound in which 2 hetero-rings are bonded by a methine group or a polymethine group, and at least one of said hetero-ring is a nitrogen-containing hetero-ring, and the one, in which both of 2 hetero-rings described above is nitrogen-containing hetero-ring, is preferable. As a substituent derived from the above-mentioned cyanine dye, for example, the one derived from Cy-based dye described in US 4,981,977, US 5,268,486, US 5,486,616 or the like; the one derived from Dy-based dye described in US 6,083,485 or the like; the one derived from HiLyte-based dye described in WO 2006/047452 or the like; the one derived from Alexa-based dye or the like are preferable. In addition, the one derived from commercially available one may be used, for example, in case of using the one derived from Cy-base dye, the one derived from Cy3, Cy3.5, Cy5, Cy5.5, Cy7 or the like, [all of them are produced from Amersham Biosciences Co. Ltd: product name]; as one derived from Dy-based dye, the one derived from DY-700, DY-701, DY-730, DY-731, DY-732, DY-734, DY-750, DY-751, DY-752, DY-776, DY-780, DY-781, DY-782 or the like; as the one derived from HiLyte-based dye, the one derived from HiLyte Fluor 555, HiLyte Fluor 647, HiLyte Fluor 680, HiLyte Fluor 750 or the like [all of them are produced from Ana Spec Co. Ltd: product name]; as the one derived from Alexa-based dye, the one derived from Alexa Fluor Dye 532, Alexa Fluor Dye 546, Alexa Fluor Dye 555, Alexa Fluor Dye 568, Alexa Fluor Dye 594, Alexa Fluor Dye 633, Alexa Fluor Dye 647, Alexa Fluor Dye 660, Alexa Fluor Dye 680, Alexa Fluor Dye 700, Alexa Fluor Dye 750 or the like [all of them are produced from Molecular Probes Co. Ltd: product name] are included as the preferable one. Among them, the one derived from Cy-based dye is preferable, particularly, among them, the one derived from Cy5 is preferable.

Also, intercalator used in the above-mentioned fluorescence detection may be the one which emits strong fluorescence by binding to nucleic acid strand, specifically, there are included, for example, acridine dye such as acridine orange; for example, ethidium compound such as ethidium bromide, ethidium homodimer-1 (EthD-1), ethidium homodimer-2 (EthD-2), ethidium bromide monoazide (EMA), dihydroethidium; for example, iodine compound such as propidium iodide, hexidium iodide; for example, 7-aminoactinomycin D (7-AAD); for example, cyanine dimer type dye such as POPO-1, BOBO-1, YOYO-1, TOTO-1, JOJO-1, POPO-3, LOLO-1, BOBO-3, YOYO-3, TOTO-3 (all of them are products name of Molecular Probes Co. Ltd); for example, cyanine monomer dye such as PO-PRO-1, BO-PRO-1, YO-PRO-1, TO-PRO-1, JO-PRO-1, PO-PRO-3, LO-PRO-1, BO-PRO-3, YO-PRO-3, TO-PRO-3, TO-PRO-5 (all of them are products name of Molecular Probes Co. Ltd): for example, SYTOX type dye such as SYBR Gold, SYBR Green I and SYBR Green II, SYTOX Green, SYTOX Blue, SYTOX Orange (all of them are products name of Molecular Probes Co. Ltd), or the like. Also, the one binding to minor group of DNA double helix [for example, 4,6-diamidino-2-phenylindole (DAPI: product name of Molecular Probes Co. Ltd,); pentahydrate (bis-benzimide) (Hoechst 33258: product name of Molecular Probes Co. Ltd); trihydrochloride (Hoechst 33342 : product name of Molecular Probes Co. Ltd); bisbenzimide dye (Hoechst 34580: product name of Molecular Probes Co. Ltd), or the like]; the one specifically binding to Adenine-Tymine (A-T) sequence [for example, acridine dye such as 9-amino-6-chloro-2-methoxyacridine(ACMA); bis-(6-chloro-2-methoxy-9-acrydinyl) spermine (acridine homodimer), for example, hydroxystilbamidine or the like], or the like can be used similarly as intercalator.

The method of the present invention is specifically carried out, for example, as described below.

That is, for example, in case of detecting the mutant DNA on human genome derived from cancer patient, PCR reaction is carried out by using human genome DNA extracted/purified by commercially available kit or the like, as sample. Sample for PCR reaction, for example, may be prepared based on the method that primer (Forward and Reverse) to amplify DNA, which is a detection target, is dissolved in 20 $\mu$L of buffer solution such as Tris buffer solution, respectively, to adjust usually 100 to 1000 nM, and 4 kinds of deoxynucleotide triphospahate(dNTPs) are dissolved to adjust usually 0.1 to 500nM respectively, and Taq DNA polymerase is dissolved to adjust 1 to 5 units, then, adding 1 ng to 100 ng of human genome DNA; PCR reaction, for example, is carried out by performing 35 to 40 cycles of reaction so that 1 cycle is specified as (1) at 93 to 98 °C for 10 to 30 seconds; (2) at 50 to 60 °C for 10 to 30 seconds; (3) at 68 to 72 °C for 1 to 3 minutes to amplify the subject single-stranded DNA. After PCR reaction, the 0.1 to 10 times amount of the subject DNA of the probe relevant to the present invention is added, and LH reaction is carried out. That is, for example, after PCR reaction, the probe relevant to the present invention is added to adjust final concentration to 100 to 500 nM, and it is carried out by 1 to 4 cycles of reaction so that 1 cycle is specified as at 90 to 100 °C for 2 to 4 minutes; at 30 to 55 °C for 1 to 30 seconds; at 65 to 75 °C for 1 to 4 minutes, thereby DNA hybrid for detection purpose can be obtained. The obtained solution is migrated, for example, by capillary-tip electrophoresis method or the like, and is detected, for example, by fluorescence detector or the like, therefore, the mutant DNA can be detected.

The present invention is further described in detail by Synthetic Example, Example, Comparative Example or the like below, however, the present invention is not limited at all by these Examples or the like.

[EXAMPLE]

Synthetic Example 1: Preparation of sample DNA having sequence of poly-T strand length polymorphism in TOMM40 gene sequence

(1) Preparation of sample DNA having various poly-T strand length polymorphism in rs10524523 marker sequence of TOMM40 gene sequence

As poly-T sequence having different strand length of rs10524523 marker sequence of TOMM40 gene sequence, 12 kinds of synthetic oligonucleotides were designed, and the complementary strands thereof were synthesized, and these were specified as sample DNA. Sequences of said synthetic oligonucleotide are shown in the following Table-1.

Synthesis of the above-mentioned nucleotide has used contract synthesis service by Sigma Genosys Co. Ltd. In addition, in following Synthesis Example and Example of the present invention, synthesis of oligonucleotide of the primer and probe, and labelling of fluorescence dye, or the like have used contract synthesis service by Sigma Genosys Co. Ltd, as well.

[Table-1]

| Sample DNA (number of poly-T in corresponding TOMM40 sequence) | Nucleotide sequence [underlined part (boldfaced type) shows microsatellite region] | SEQ ID NO |
|---|---|---|
| Q10A (poly=T10) | taggcattcgaagccagcccgggcaacatggtgagaccccatctc**aaaaaaaaaa**gccagatgcaatggctcatg | SEQ ID NO: [1] |
| Q15A (poly-T=15) | taggcattcgaagccagcccgggcaacatggtgagaccccatctc**aaaaaaaaaaaaaaa**gccagatgcaatggctcatg | SEQ ID NO: [2] |
| Q16A (poly-T=16) | taggcattcgaagccagcccgggcaacatggtgagaccccatctc**aaaaaaaaaaaaaaaa**gccagatgcaatggctcatg | SEQ ID NO: [3] |
| Q18A (poly-T=18) | taggcattcgaagccagcccgggcaacatggtgagaccccatctc**aaaaaaaaaaaaaaaaaa**gccagatgcaatggctcatg | SEQ ID NO: [4] |
| Q19A (poly-T=19) | taggcattcgaagccagcccgggcaacatggtgagaccccatctc**aaaaaaaaaaaaaaaaaaa**gccagatgcaatggctcatg | SEQ ID NO: [5] |
| Q20A (poly-T=20) | taggcattcgaagccagcccgggcaacatggtgagaccccatctc**aaaaaaaaaaaaaaaaaaaa**gccagatgcaatggctcatg | SEQ ID NO: [6] |
| Q21A (poly-T=21) | taggcattcgaagccagcccgggcaacatggtgagaccccatctc**aaaaaaaaaaaaaaaaaaaaa**gccagatgcaatggctcatg | SEQ ID NO: [7] |
| Q22A (poly-T=22) | taggcattcgaagccagcccgggcaacatggtgagaccccatctc**aaaaaaaaaaaaaaaaaaaaaa**gccagatgcaatggctcatg | SEQ ID NO: [8] |
| Q25A (poly-T=25) | taggcattcgaagccagcccgggcaacatggtgagaccccatctc**aaaaaaaaaaaaaaaaaaaaaaaaa**gccagatgcaatggctcatg | SEQ ID NO: [9] |
| Q28A (poly-T=28) | taggcattcgaagccagcccgggcaacatggtgagaccccatctc**aaaaaaaaaaaaaaaaaaaaaaaaaaaa**gccagatgcaatggctcatg | SEQ ID NO: [10] |

(continued)

| Sample DNA (number of poly-T in corresponding TOMM40 sequence) | Nucleotide sequence [underlined part (boldfaced type) shows microsatellite region] | SEQ ID NO |
|---|---|---|
| Q29A (poly-T=29) | taggcattcgaagccagcccgggcaacatggtgagacccc atctc**aaaaaaaaaaaaaaaaaaaaaaaaaaaaa**gcc agatgcaatggctcatg | SEQ ID NO: [11] |
| Q30A (poly-T=30) | taggcattcgaagccagcccgggcaacatggtgagacccc atctc**aaaaaaaaaaaaaaaaaaaaaaaaaaaaaa**gc cagatgcaatggctcatg | SEQ ID NO: [12] |

(2) Extension of sample DNA and double-strand formation

Further, from the following operation experiment, 12 kinds of single-stranded DNA were used as template, and double-stranded DNA in which 3'-terminal side of respective sequence was extended by 51 nucleotides was synthesized.

That is, first of all, the following sequence (Fx sequence) having 71 nucleotides [SEQ ID NO: 13] which is the sequence on the sequence of TOMM40 gene comprising complementary strand (underlined part in the following sequence) of 20 nucleotides from 3'-terminal of these 12 kinds of sequences to just before poly-T was synthesized. gacctcaagctgtcctcttgccccagccctccaaagcattgggattactggcatgagccattgcatctggc

Subsequently, the above-mentioned 12 kinds of sample DNA were respectively used as direct template DNA, and over-lapping extension reaction with Fx sequence was carried out. That is, after 1.0 μL of 2μ M Fx sequence (final concentration 200 nM) was added to 1.0 μL of 5μ M each sample DNA (final concentration 500 nM), 0.04 μL of AccuPrime Taq polymerase High Fidelity (produced by Invitron Corporation) and 1.0 μL of 10x buffers attached on the product were added, and furthermore, deionized sterilized water ($ddH_2O$) was added to prepare 10 μL of reaction solution. Subsequently, by using 10 μL of said solution, process (1 cycle) until annealing and extended strand reaction under the following reaction condition by using DNA thermal cycler (DNA Engine PTC200 : MJ Research Co. Ltd) was carried out.

(Condition for the reaction of annealing and extension)

| | |
|---|---|
| 105 °C: | hot lid; |
| 94 °C: | 2 minutes; |
| 55 °C: | 15 seconds; |
| 68 °C: | 4 minutes; |

Thus, the double-stranded DNAs were obtained by performing double-strand formation by the extension of the above-mentioned each 12 kinds of sample DNAs. Schematic diagram of said reaction is shown as follows:

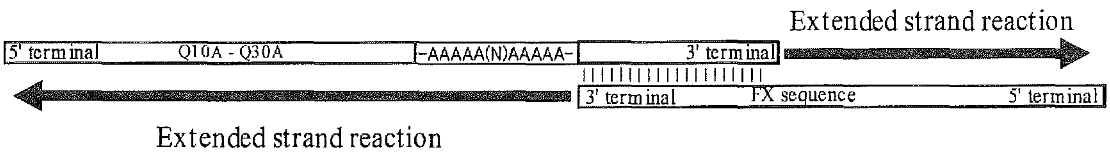


(3) Amplification of sample DNA

Subsequently, after each of the above-mentioned double-stranded DNA sample was diluted 1000 times, 1 μL of solution thereof was used as template for amplifying PCR, and PCR reaction was carried out by using AccuPrime Taq polymerase system (kit for PCR reaction: manufactured by Invitrogen Co. Ltd). That is, first of all, according to the product protocol attached on the kit, by using 2.0 μL of buffer solution for PCR reaction and 0.5 μL of AccuPrime Taq enzyme which are attached on the kit, 1.0 μL of each 10μM primer (Forward: gacctcaagctgtcctcttgcc [SEQ ID NO: 14], Reverse: taggcattcgaagccagcccg [SEQ ID NO: 15]) and 16.5 μL of $ddH_2O$, 20.0 μL of reaction solution for PCR was prepared. By using this reaction solution for PCR, 20 cycle of PCR reaction was carried out under the following reaction condition using DNA thermal cycler (DNA Engine PTC200) manufactured by MJ Research Co. Ltd.

*Condition for PCR reaction

| | |
|---|---|
| Thermal denaturation: | 95 °C, 15 seconds; |
| Annealing: | 55 °C, 15 seconds; |
| Polymerization reaction: | 68 °C, 47 seconds; |

As for double-stranded DNA thus prepared, the one synthesized from Q10A was specified as Fx/Q10A; the one synthesized from Q15A was specified as Fx/Q15A; the one synthesized from Q16A was specified as Fx/Q16A; the one synthesized from Q18A was specified as Fx/Q18A; the one synthesized from Q19A was specified as Fx/Q19A; the one synthesized from Q20A was specified as Fx/Q20A; the one synthesized from Q21A was specified as Fx/Q21A; the one synthesized from Q22A was specified as Fx/Q22A; the one synthesized from Q25A was specified as Fx/Q25A; the one synthesized from Q28A was specified as Fx/Q28A; the one synthesized from Q29A was specified as Fx/Q29A; the one synthesized from Q30A was specified as Fx/Q30A, respectively. Single-stranded DNA sequence of one side in these double-stranded DNA is shown in Table-2.

[Table-2]

| Sample DNA (poly-T strand length) | Sequence [underlined part (boldfaced type) shows microsatellite region] |
|---|---|
| Fx/Q10A (poly-T=10) [SEQ ID NO: 16] | taggcattcgaagccagcccgggcaacatggtgagaccccatctc**aaaaaaaa**<br>**aa**gccagatgcaatggctcatgccagtaatcccaatgctttggagggctggggca<br>agaggacagctt<br>gaggtc |
| Fx/Q15A (poly-T=15) [SEQ ID NO: 17] | Taggcattcgaagccagcccgggcaacatggtgagaccccatctc**aaaaaaaa**<br>**aaaaaaa**gccagatgcaatggctcatgccagtaatcccaatgctttggagggctg<br>gggcaagaggacagcttgaggtc |
| Fx/Q16A (poly-T=16) [SEQ ID NO: 18] | Taggcattcgaagccagcccgggcaacatggtgagaccccatctc**aaaaaaaa**<br>**aaaaaaaa**gccagatgcaatggctcatgccagtaatcccaatgctttggagggct<br>ggggcaagaggacagcttgaggtc |
| Fx/Q18A (poly-T=18) [SEQ ID NO: 19] | Taggcattcgaagccagcccgggcaacatggtgagaccccatctc**aaaaaaaa**<br>**aaaaaaaaaa**gccagatgcaatggctcatgccagtaatcccaatgctttggagg<br>gctggggcaagaggacagcttgaggtc |
| Fx/Q19A (poly-T=19) [SEQ ID NO: 20] | Taggcattcgaagccagcccgggcaacatggtgagaccccatctc**aaaaaaaa**<br>**aaaaaaaaaaa**gccagatgcaatggctcatgccagtaatcccaatgctttggag<br>ggctggggcaagaggacagcttgaggtc |
| Fx/Q20A (poly-T=20) [SEQ ID NO: 21] | Taggcattcgaagccagcccgggcaacatggtgagaccccatctc**aaaaaaaa**<br>**aaaaaaaaaaaa**gccagatgcaatggctcatgccagtaatcccaatgctttgga<br>gggctggggcaagaggacagcttgaggtc |
| Fx/Q21A (poly-T=21) [SEQ ID NO: 22] | taggcattcgaagccagcccgggcaacatggtgagaccccatctc**aaaaaaaa**<br>**aaaaaaaaaaaaa**gccagatgcaatggctcatgccagtaatcccaatgctttgg<br>agggctggggcaagaggacagcttgaggtc |
| Fx/Q22A (poly-T=22) [SEQ ID NO: 23] | taggcattcgaagccagcccgggcaacatggtgagaccccatctc**aaaaaaaa**<br>**aaaaaaaaaaaaaa**gccagatgcaatggctcatgccagtaatcccaatgctttg<br>gagggctggggcaagaggacagcttgaggtc |
| | |

(continued)

| Sample DNA (poly-T strand length) | Sequence [underlined part (boldfaced type) shows microsatellite region] |
|---|---|
| Fx/Q25A (poly-T=25) [SEQ ID NO: 24] | taggcattcgaagccagcccgggcaacatggtgagaccccatctc**aaaaaaaaaaaaaaaaaaaaaaaaa**gccagatgcaatggctcatgccagtaatcccaatgctttggagggctggggcaagaggacagcttgaggtc |
| Fx/Q28A (poly-T=28) [SEQ ID NO: 25] | taggcattcgaagccagcccgggcaacatggtgagaccccatctc**aaaaaaaaaaaaaaaaaaaaaaaaaaaa**gccagatgcaatggctcatgccagtaatcccaatgctttggagggctggggcaagaggacagcttgaggtc |
| Fx/Q29A (poly-T=29) [SEQ ID NO: 26] | taggcattcgaagccagcccgggcaacatggtgagaccccatctc**aaaaaaaaaaaaaaaaaaaaaaaaaaaaa**gccagatgcaatggctcatgccagtaatcccaatgctttggagggctggggcaagaggacagcttgaggtc |
| Fx/Q30A (poly-T=30) [SEQ ID NO: 27] | taggcattcgaagccagcccgggcaacatggtgagaccccatctc**aaaaaaaaaaaaaaaaaaaaaaaaaaaaaa**gccagatgcaatggctcatgccagtaatcccaatgctttggagggctggggcaagaggacagcttgaggtc |

Example 1: Discrimination/detection of poly-T strand length polymorphism of TOMM40 gene according to the method of the present invention

(1) Preparation of the probe for loop hybrid reaction (LH probe)

LH probe was designed so that it does not bind to the microsatellite region in DNA described in Table-2, and binds to the both sides thereof. That is, it was specified as LH probe that bound the complementary strand of 19 nucleotides from next of 5'-terminal in the microsatellite region of the complementary sequence of single-stranded DNA in the above-mentioned Table-2., and the complementary strand of 71 nucleotides from next of 3'-terminal side, Specifically, the following one (cy5delT0) was used:

(cy5delT0;90mer, T0=deleted poly T [SEQ ID NO: 28])

gacctcaagc tgtcctcttg ccccagccct ccaaagcatt gggattactg gcatgagcca ttgcatctgg c(T0)gagatgggg tctcaccatg.

When the above-mentioned LH probe forms the hybrid with the target DNA, said hybrid forms the loop structure in the region not hybridized with LH probe sequence. That is, the microsatellite region on the target DNA forms the loop structure. Specifically, it is considered that the following loop structure is formed on the target DNA sequence.

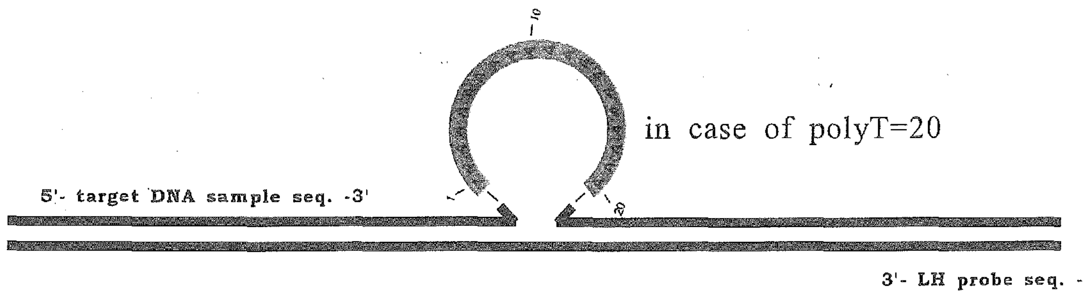


(2) LH reaction

Among the above-mentioned 2, Fx/Q10A, Fx/Q15A, Fx/Q16A, Fx/Q18A, Fx/Q19A, Fx/Q20A, Fx/Q21A, Fx/Q22A and Fx/Q25A were used as sample, and LH reaction was carried out. That is, after LH probe synthesized in (1) (ID.= cy5de1T0) was added to 4.5 μL of PCR reaction solution including the above-mentioned sample obtained in the above-mentioned Synthetic Example 1 (3), respectively, to adjust the final concentration to 200 nM, 1 cycle of LH reaction was carried out under the following reaction condition by using DNA thermal cycler (DNA Engine PTC200, manufactured from MJ Research Co. LTD) to obtain LH reaction product.

* LH reaction condition

Sample solution: 4.5 μl;
LH probe: 0.5 μl (2 μM);

| | |
|---|---|
| 105 °C: | hot lid; |
| 95 °C: | 2 minutes: |
| 55 °C: | 30 seconds; |
| 68 °C: | 4 minutes; |
| 4 °C: | Reaction stop; |

(3) Separation/Detection of LH reaction product

After 1.5 μl of gel loading buffer was respectively added to each 1.5 μl of 9 kinds of LH reaction products obtained in the above-mentioned (2), electrophoresis was carried out by using 10% of nondenaturing polyacrylamide gel. As a molecular weight marker, 1.5μl of 100 bp ladder for size marker (produced by Promega Co. Ltd) was used, and electrophoresis was carried out by loading to the same gel. As polyacrylamide gel, 7cm x 7cm of compact gel was used (Compact gel C10L, produced by Atto Co. Ltd), and Tris-glycine buffer solution (37.5 mM: Tris; 288 mM: Glycine) was used as migrating buffer solution, and electrophoresis was carried out at room temperature with small size electrophoresis instrument (AE-7300 Compact PAGE: manufactured by Atto Co. Ltd).

After migrating, it was dyed with Cyber Green I (F0513 produced by Takara Bio Co. Ltd) for 10 min, and after washing with water, and was detected by using laser imaging scanner (STORM 860, manufactured by Amersham Co. Ltd), under the condition that excitation wavelength is 450 nm, and detection scanner is 520LP. The obtained result is shown as Fig. 1. In the figure, lane 10 shows the result of migrating the LH reaction product of Fx/Q10A; lane 15 shows the result of migrating the LH reaction product of Fx/Q15A; lane 16 shows the result of migrating the LH reaction product of Fx/Q16A; lane 18 shows the result of migrating the LH reaction product of Fx/Q18A; lane 19 shows the result of migrating the LH reaction product of Fx/Q19A; lane 20 shows the result of migrating the LH reaction product of Fx/Q20A; lane 21 shows the result of migrating the LH reaction product of Fx/Q21A; lane 22 shows the result of migrating the LH reaction product of Fx/Q22A; lane 25 shows the result of migrating the LH reaction product of Fx/Q25A, respectively.

As apparent from the results shown in Fig 1, LH reaction is carried out by using the probe designed so that loop includes all of the microsatellite region, and from the result that the obtained LH reaction product was separated/detected by using acrylamide gel electrophoresis, it was found that band derived from non-specific reaction product can hardly been found, and, even TOMM40 gene having poly-T sequence having different strand length can be sufficiently discriminated/detected.

<u>Comparative Example 1 Discrimination/Detection of poly-T strand length of TOMM40 gene by LH probe including a part of microsatellite region</u>

(1) Preparation of LH Probe

The probe was designed so that the complementary DNA with DNA described in Table-2 is specified as detection target, and it binds to a part of the microsatellite region in said detection target DNA, and does not bind to remainder part thereof. That is, LH probe is specified as the one which bound the complementary strand of 55 nucleotides from next of 5'-terminal in the microsatellite region of single-stranded DNA in the above-mentioned Table-2, and PolyA of 10 nucleotides and the complementary strand of 20 nucleotides from next of 3'-terminal. Specifically, the following sequence (cy5Q10A) was used.

(cy5Q10A; 75mer [SEQ ID NO: 29])
taggcattcgaagccagcccgggcaacatggtgagaccccatctcaaaaaaaaaagccagatgcaatggctcatg

It should be noted that, when said probe formed the hybrid with target DNA, it is considered that region in which target DNA is not hybridized with probe sequence, that is, a part of the microsatellite region on target DNA and nucleotide sequence other than the microsatellite region form loop structure.

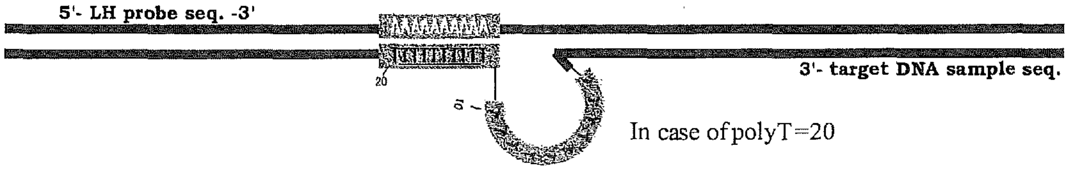


(2) LH Reaction and Separation/Detection of LH reaction product

By the same method as Example 1(2) and (3) except for using the above-mentioned probe, discrimination/detection of poly-T strand length polymorphism on Fx/Q10A, Fx/Q15A, Fx/Q16A, Fx/Q18A, Fx/Q19A, Fx/Q20A, Fx/Q21A, Fx/Q22A, and Fx/Q25A were carried out.

This result is shown in Fig. 2. In the figure, lane 10 shows the result of migrating LH reaction product of Fx/Q10A; lane 15 shows the result of migrating LH reaction product of Fx/Q15A; lane 16 shows the result of migrating LH reaction product of Fx/Q16A; lane 18 shows the result of migrating LH reaction product of Fx/Q18A; lane 19 shows the result of migrating LH reaction product of Fx/Q19A; lane 20 shows the result of migrating LH reaction product of Fx/Q20A; lane 21 shows the result of migrating LH reaction product of Fx/Q21A; lane 22 shows the result of migrating LH reaction product of Fx/Q22A; lane 25 shows the result of migrating LH reaction product of Fx/Q25A respectively.

As shown in Fig. 2, when using LH probe by the conventional LH method, many signal bands of non-specific reaction products are found. In particular, in case that as the number of repetitive sequence of poly-T region, 15 and 16 was used, band of no-specified reaction product is strongly detected (shown clearly with an arrow mark in Figure). Therefore, it was found that it is difficult to specify LH reaction product by said method. It should be noted that, the reason why the non-specific reaction product is produced, is not clear, however, it was estimated that due to containing the complementary sequence in a part of the microsatellite region so that the probe binds to a part of the microsatellite region, such linkage error in which the repetitive sequence of the complementary strand in the probe binds to another repetitive sequence than the set repetitive sequence, or the like, was caused, and a plurality of non-specific reaction products were produced.

Therefore, from the result of Fig. 1 and Fig. 2, it was found that discrimination/detection of poly-T strand length polymorphism which is difficult in case of using the conventional LH probe, can be clearly discriminated/detected in case of using the probe of microsatellite region deleted type LH probe relevant to the preset invention (Fig. 1).

It should be noted that, in Fig. 2, in case that number of repetitive sequence in poly-T region is 10 (lane 10), band of reaction product cannot be found, however, this is because LH probe hybridizes completely complementarily with target DNA and loop structure is not formed.

Example 2 Discrimination/Detection of poly-T strand length polymorphism of TOMM40 gene by the method of the present invention in which length strand poly-T polymorphism is specified as detection target

Discrimination/detection of poly-T strand length polymorphism in TOMM40 gene sequence was carried out by the same method as Example 1 (2) and (3) except for using Fx/Q28A, Fx/Q29A, or Fx/Q30A as sample. This Result is shown in Fig. 3-a. In the Figure, lane 28 represents the result of migrating LH reaction product of Fx/Q28A; lane 29 represents the result of migrating LH reaction product of Fx/Q29A; lane 30 represents the result of migrating LH reaction product of Fx/Q30A, respectively.

Comparative Example 2 Discrimination/detection of poly-T strand length polymorphism of TOMM40 gene by LH probe including a part of the microsatellite region in case of specifying length strand poly-T polymorphism as detection target

(1) Preparation of LH Probe

LH probe (cy5Q5A) in which 10 nucleotides of polyA in LH probe (cy5Q10A) used in Comparative Example 1 is changed to 5 nucleotides of polyA, and LH probe (cy5Q25A) in which 10 nucleotides of polyA is changed to 25 nucleotides of polyA were synthesized, respectively. Specifically, the one including the following sequence was synthesized.

(cy5Q5A; 70mer [SEQ ID NO: 30])

taggcattcgaagccagcccgggcaacatggtgagaccccatctcaaaaagccagatgcaatggctcatg

(cy5Q25A ; 90mer [SEQ ID NO: 31])

taggcattcgaagccagcccgggcaacatggtgagaccccatctcaaaaaaaaaaaaaaaaaaaaaaaaagcca

gatgcaatggctcatg

It should be noted that, when these probes formed the hybrid with target DNA, similarly to Comparative Example 1, it is considered that, loop structure is formed by a part of the microsatellite region on target DNA and the nucleotide chain other than the microsatellite region.

(2) LH reaction and Separation/Detection of LH reaction product

Discrimination/detection of poly-T strand length polymorphism in TOMM40 gene sequence was carried out by the same method as Example 1 (2) and (3) except for using Fx/Q28A, Fx/Q29A, or Fx/Q30A as sample, and the above-mentioned cy5Q5A or cy5Q25A as probe. The result using cy5Q5A as probe was shown in Fig. 3-b; the result using cy5Q25A as probe was shown in Fig. 3-c respectively.

As shown in Fig. 3-a, it was understood that in case of using LH probe (cy5de1T0) relevant to the present invention, sample having poly-T which contains 28 nucleotides, 29 nucleotides, or 30 nucleotides can be clearly separated, and at the same time non-specific reaction product is hardly found. On the other hand, in case of using cy5Q5A, a plurality of signal bands hindering the decision of detection result was much detected, and it was found that it is difficult to discriminate/detect the sequence polymorphism sample. In addition, in case of using cy5Q25A, numerous non-specific reactions are found, and thus it was very difficult to confirm the objective LH reaction product.

As apparent from these results, a lot of the non-specific reaction products are found according to the conventional LH method, and thus, it was difficult to specify the objective LH reaction product, however, it was found that according to the present invention, discrimination/detection can be clearly carried out.

Example 3 Discrimination/detection of CAG-repeat polymorphism in Androgen Receptor (AR) gene sequence

(1) Preparation of human genome DNA derived from human blood and cloning sample

A plurality of samples derived from human blood are prepared, after treating 2 ml of whole blood with proteinase K at 70 °C, for 10 minutes according to QIAamp DNA Blood Midi Kit(Qiagen Co. Ltd), ethanol was added.
Further, supernatant after centrifugal separation was subjected to QIAmp Midi column, and, human genome DNA was extracted. Among 30 μL of each DNA extraction liquid obtained here, 1 μL was used as template, and PCR reaction was carried out by using AccuPrime Taq polymerase system (PCR reaction kit, manufactured by Invitrogen Co. Ltd). That is, first of all, according to product protocol attached on the kit, 2.0 μL of PCR reaction buffer solution, 0.5 μL of AccuPrime Taq enzyme, which are attached on the kit, 1.0 μL of each 10 μM primers (Forward: agcgtgcgcgaagtgatcca [SEQ ID NO:: 32], Reverse: atgggcttggggagaaccat [SEQ ID NO:: 33]) and 16.5 μL of dd$H_2$ O were used, and 20.0 μL of reaction solution for PCR was prepared. By using this sample for PCR, by using DNA thermal cycler (DNA Engine PTC200, manufactured by MJ Research Co. Ltd), 30 cycles of PCR reaction was carried out under the following reaction condition.

*PCR reaction condition

| | |
|---|---|
| Thermal denaturation: | at 95 °C for 15 seconds; |
| Annealing: | at 55 °C for 15 seconds; |
| Polymerization reaction: | at 68 °C for 47 seconds; |

According to these series of PCR reactions, each of PCR product obtained from a plurality of starting materials was introduced into a plasmid vector pGEM-T easy by TA cloning method, using pGEM-T Vector System (Promega Co. Ltd). That is, to 3.0 μL of PCR amplified products respectively, 1.0 μL of pGEM-T Easy Vector (produced by Promega Co. Ltd) and 5.0 μL of Rapid Ligation Buffer of DNA Ligation Kit (produced by Promega Co. Ltd) and 1.0 μL of T4 ligase were added to be total amount by 10.0 μL and it was incubated at room temperature for 60 min, and thus, recombinant DNA was obtained.

Thereafter, by using E. coli JM109 Competent Cells (produced by Toyobo Co. Ltd), according to the product protocol thereof, transformation of E. coli JM109 Competent Cells was carried out using the recombinant DNA obtained above at 42 °C for 45 seconds. Thereafter, the obtained transformant was cultivated in plate in LB-agar medium including 100 μg /ml of ampicillin, 0.2 mM isopropyl-β-thiogalacropyranoside (IPTG), 40 μg /ml of X-Gal, at 37 °C for 16 hrs. After cultivation, white colony in the culture medium was picked up, then the tranformant for each clone in which the recombinant DNA inserted the objective DNA fragment was introduced, was obtained. Thereafter, by using plasmid extraction kid (QIAprep Spin Miniprep) of QIAGEN Co. Ltd, process up to extraction/purification of DNA was carried out. That is, transformant for each clone which was propagated overnight in 5 ml of 100 μg /ml ampicillin-containing LB liquid medium was harvested by centrifugation, and after bacteriolysis by alkali method, it was neutralized with potassium acetate acidic liquid, and plasmid DNA was purified by the purified column attached on the kit from supernatant liquid after centrifuging them.

(2) Confirmation of polymorphism sequence DNA on AR gene exon 1

Next, by using candidate clone which is expected to include the sequence of a plurality of kinds of gene polymorphism cloned in the above, and by Big Dye Terminator kit (manufactured by Applied Bio-system Co. Ltd), according to the product protocol, sequence analysis was carried out based on the following procedure.

That is, $ddH_2O$ was added to the mixture including 2 μL (100 ng) of sample DNA (each clone), 1 μL (5 pmol) of T7 promoter primer, and 8 μL of the mixture of premix including enzyme, dNTPs, reaction buffer and fluorescence dye, so as to adjust the total amount to 20 μL, and by using DNA thermal cycler (DNA Engine PTC200, manufactured by MJ Research Co. Ltd), 30 cycles of sequence reaction was carried out under the following reaction condition.

at 96 °C 2 minutes → to (at 96 °C for 10 seconds to at 50 °C for 5 seconds to at 60 °C for 2 minutes) ×25 to → 4 °C;

After purifying the obtained sequence reaction product using gel filtration column (manufactured by QIAGEN Co. Ltd), by using Sequencer (3130 Genetic Analyzer, manufactured by Applied Biosystems Co. Ltd), according to procedure manual attached to instrument, sequence decoding of all candidate sequences were completed.

As a result, it was confirmed that sample DNA including 6 kinds of the following Table-3 gene polymorphism sequence on AR gene Exon 1 can be produced. That is, it was confirmed that 6 kinds of sample DNA having different length in the microsatellite region was prepared. These are referred as Sample M6, Sample M18, Sample M7, Sample M25, Sample M2 respectively.

[Table-3]

| Sample DNA (Repeat number of CAG in PCR product) | Nucleotide sequence [underlined part (boldfaced type) shows microsatellite region different in length] |
|---|---|
| M6 (26) [SEQ ID NO: 34] | agcgtgcgcgaagtgatccagaacccgggccccaggcacccagaggccgcgagcgcagca cctcccggcgccagtttgctgctgctg**cagcagcagcagcagcagcagcagcagcag cagcagcagcagcagcagcagcagcagcagcagcagcagcag**caagagactagcc ccaggcagcagcagcagcagcagggtgaggatggttctccccaagcccatcgaagaggccc cagaggctaggtggtgcaggatgaggaacagccaccttcacagc |
| M18 (24) [SEQ ID NO: 35] | agcgtgcgcgaagtgatccagaacccgggccccaggcacccagaggccgcgagcgcagca cctcccggcgccagtttgctgctgctg**cagcagcagcagcagcagcagcagcagcag cagcagcagcagcagcagcagcagcagcagcagcag**caagagactagccccaggc agcagcagcagcagcagggtgaggatggttctccccaagcccatcgaagaggccccagagg ctaggtggtgcaggatgaggaacagccaccttcacagc |

(continued)

| Sample DNA (Repeat number of CAG in PCR product) | Nucleotide sequence [underlined part (boldfaced type) shows microsatellite region different in length] |
|---|---|
| M7 (23) [SEQ ID NO: 36] | agcgtgcgcgaagtgatccagaacccgggccccaggcacccagaggccgcgagcgcagca<br>cctcccggcgccagtttgctgctgctg**cagcagcagcagcagcagcagcagcagcagcag**<br>**cagcagcagcagcagcagcagcagcagcagcagcag**caagagactagccccaggcagc<br>agcagcagcagcagggtgaggatggttctccccaagcccatcgtagaggccccacaggctac<br>ctggtcctggatgaggaacagcaaccttcacagc |
| M25 (22) [SEQ ID NO: 37] | agcgtgcgcgaagtgatccagaacccgggccccaggcacccagaggccgcgagcgcagca<br>cctcccggcgccagtttgctgctgctg**cagcagcagcagcagcagcagcagcagcagcag**<br>**cagcagcagcagcagcagcagcagcagcagcag**caagagactagccccaggcagcagc<br>agcagcagcagggtgaggatggttctccccaagcccatcgtagaggccccacaggctacctg<br>gtcctggatgaggaacagcaaccttcacagc |
| M2 (21) [SEQ ID NO: 38] | agcgtgcgcgaagtgatccaraacccgggccccaggcacccagaggccgcgagcgcagca<br>cctcccggcgccagtttgctgctgctg**cagcagcagcagcagcagcagcagcagcagcag**<br>**cagcagcagcagcagcagcagcagcagcag**caagagactagccccaggcagcagcagc<br>agcagcagggtgaggatggttctccccaagcccatcgtagaggccccacaggctacctggtc<br>ctggatgaggaacagcaaccttcacagc |

(3) Preparation of LH probe

LH probe was designed so that it does not bind to the microsatellite region in DNA described in Table-3 but binds to both sides thereof. That is, the one binding the complementary strand of the nucleotide sequence of 17 nucleotides from next of 2 nucleotides of 5'-terminal of microsatellite region in single-stranded DNA in the above-mentioned Table-3, and the complementary strand of the nucleotide sequence of 18 nucleotides from next of 2 nucleotides of 3'-terminal side, and the complementary strand of the nucleotide sequence of 29 nucleotides from nucleotide from next of 14 nucleotides further therefrom, was designed as LH probe. Specifically, the one fluorescence-modified with Cy5 at 5'-terminal of the following nucleotide sequence (Cy5ARdelCTG0-20R) was synthesized as probe:

(Cy5ARdelCTG0-20R: 64mer [SEQ ID NO: 39]).

atgggcttggggagaaccatcctcacccttgcctggggctagtctctgcagcagcaaactggcg

When the above-mentioned probe formed the hybrid with targeting DNA, the region not hybridized with said probe sequence, that is, the microsatellite region on DNA forms the loop structure, and further, the microsatellite region which is positioned from the microsatellite region in single-stranded DNA in the Table-3 toward the 3'-terminal direction by 19 nucleotides downstream, forms the loop structure.

(4) microsatellite region deleted-type LH probe reaction

By using each sample DNA above-mentioned in Table-3 as sample, and using AccuPrime Taq polymerase system (PCR reaction kit, manufactured by Invitorogen Co. Ltd), PCR reaction was carried out. That is, first of all, according to the product protocol attached on the kit, by using 2.0 μL of PCR reaction buffer solution, 0.5 μL of AccuPrime Taq enzyme, which are attached on the kit, 1.0 μL of each 10 μM primers (Forward: agcgtgcgcgaagtgatcca [SEQ ID NO: 32] and Reverse: atgggcttggggagaaccat [SEQ ID NO: 33]), and 16.5 μL of $ddH_2O$, 20.0 μL of PCR reaction solution was prepared. Thereafter, 2 pg of each sample was added to 20 μL of PCR reaction solution, it was specified as the sample for PCR reaction. By using this sample for PCR reaction, and using DNA thermal cycler (DNA Engine PTC200) manufactured by MJ Research Co. Ltd, 30 cycles of PCR reaction was carried out under the following reaction condition.

*PCR reaction condition

| | |
|---|---|
| Thermal denaturation: | 95 °C, 15 seconds; |
| Annealing: | 55 °C, 15 seconds; |
| Polymerization reaction: | 68 °C, 47 seconds; |

After completing the reaction, LH probe (ID. = Cy5ARdelCTG0-20R) was added to PCR reaction solution to adjust the final concentration to 200 nM, and by using DNA thermal cycler (DNA Engine PTC200, manufactured by MJ Research Co. Ltd), 1 cycle reaction was carried out under the following reaction condition.

*LH reaction condition

| | |
|---|---|
| PCR reaction product: 4.5 μl; | |
| LH probe: | 0.5 μl (2 μM); |
| 105 °C: | hot lid; |
| 95 °C: | 2 minutes; |
| 55 °C: | 30 seconds: |
| 68 °C: | 4 minutes; |
| 4 °C: | reaction stop; |

(5) Separation/Detection of the microsatellite region deleted-type LH probe reaction product

To each 1.5 μl of 5 kinds of the reaction products obtained in the above-mentioned (4), 1.5 μl of gel loading buffer solution was added, respectively, and electrophoresis was carried out by using 10 % of non-denaturation polyacrylamide gel. In addition, by using 1.5 μl of 100 bp ladder for size marker (produced by Promega Co. Ltd) as a molecular weight marker, and loading this to the same gel, electrophoresis was carried out. As polyacrylamide gel, 7 cm x 7 cm of Compact gel (Compact gel C10L, produced by Atto Co..Ltd) was used. As migration buffer solution, Tris-Glycine buffer solution (37.5 mM: Tris, 288 mM: Glycine) was used, and migrating was carried out with compact electrophoresis equipment (manufactured by Atto Co. Ltd, AE-7300 Compact PAGE) at room temperature.

After migrating, by using Laser Imaging Scanner (manufactured by Amersham Co. Ltd, STORM 860), fluorescence detection (excitation wave length: 635 nm, detection filter: 650LP) was carried out. The obtained results were shown in Fig. 4. It should be noted that, in figure, lane 26 shows the migration result of LH reaction product of sample M6; lane 24 shows the migration result of LH reaction product of sample M18; lane 23 shows the migration result of LH reaction product of sample M7; lane 22 shows the migration result of LH reaction product of sample M25; lane 21 shows the migration result of LH reaction product of sample M2.

As apparent from the result of Fig. 4, the result that LH reaction product obtained by using the above-mentioned Cy5ARdelCTG0-20R probe was separated/detected by electrophoresis with acrylamide polymerized gel, showed that non-specific reaction product can hardly be found, therefore, it was shown that each sample DNA having different CAG-repeat can be separated/detected.

<u>Comparative Example 3 Discrimination/detection of CAG-repeat polymorphism in AR gene sequence by LH probe including a part of the microsatellite region</u>

(1) Preparation of LH probe

LH probe was designed so that it does not bind to a part of microsatellite region in DNA described in Table-3, and becomes complementary to residual part. That is, LH probe was designed such that the complementary strand of 19 nucleotides from next of 5'-terminal of the microsatellite region of the single-stranded DNA in the above-mentioned Table-3, and CAG-repeat 36 nucleotides, and the complementary strand of 20 nucleotides from next of 3'-terminal of the microsatellite region of the single-stranded DNA in the above-mentioned Table-3, and from there, further, the complementary sequence of nucleotide sequence of 29 nucleotide from nucleotide next of 14 nucleotides, were bound by this order. Specifically, the one which 5'-terminal of the following sequence (Cy5ARdelCTG12-20R) was fluorescence-modified with Cy5 was synthesized as probe.

(Cy5ARdelCTG12-20R; 104mer [SEQ ID NO: 40])

atgggcttggggagaaccatcctcacccttgcctggggctagtctcttgctgctgctgctgctgctgctgctgctg ctgctgcagcagcagcaaact-ggcg

It should be noted that, when said probe forms the hybrid with target DNA, it is considered that the region not hybridized with said probe sequence, that is, a part of the microsatellite region on DNA forms loop structure in the boundary region of repetitive sequence and non-repetitive sequence on the target DNA sequence.

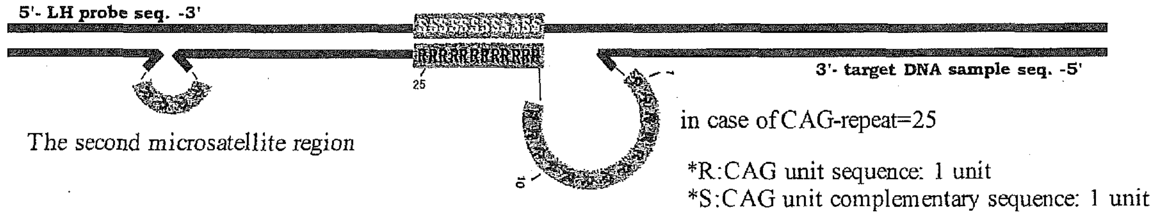


(2) LH reaction and Separation/Detection of LH reaction product

Discrimination/detection of CAG-repeat polymorphism on AR gene exon 1 was carried out by the same method as Example 3 except for using the above-mentioned probe. The result is shown in Fig. 5. As sample DNA, 5 kinds of samples shown in the above-mentioned Table-3, and 3 kinds of samples (Sample M34, Sample M15, Sample U7) having different number of CAG-repeat sequences shown in Table-4, were used. It should be noted that, these 3 kinds of samples were prepared by the same method as Example 3(1).

[Table-4]

| Sample DNA (Repeat number of CAG in PCR product) | Nucleotide sequence [underlined part (boldfaced type) shows microsatellite region] |
|---|---|
| M34 (28) [SEQ ID NO: 41] | agcgtgcgcgaagtgatccagaacccgggccccaggcacccagaggccgcgagcgca<br>gcacctcccggcgccagtttgctgctgctg**cagcagcagcagcagcagcagcagcag**<br>**cagcagcagcagcagcagcagcagcagcagcagcagcagcagcagcagcagcag**<br>**cag**caagagactagccccaggcagcagcagcagcagcagggtgaggatggttctcccc<br>aagcccatcgaagaggccccagaggctaggtggtgcaggatgaggaacagccaccttc<br>acagc |
| M15(27) [SEQ ID NO: 42] | agcgtgcgcgaagtgatccagaacccgggccccaggcacccagaggccgcgagcgca<br>gcacctcccggcgccagtttgctgctgctg**cagcagcagcagcagcagcagcagcag**<br>**cagcagcagcagcagcagcagcagcagcagcagcagcagcagcagcagcagcag**<br>caagagactagccccaggcagcagcagcagcagcagggtgaggatggttctccccaag<br>cccatcgaagaggccccagaggctaggtggtgcaggatgaggaacagccaccttcaca<br>gc |
| U7 (25) [SEQ ID NO: 43] | agcgtgcgcgaagtgatccagaacccgggccccaggcacccagaggccgcgagcgca<br>gcacctcccggcgccagtttgctgctgctg**cagcagcagcagcagcagcagcagcag**<br>**cagcagcagcagcagcagcagcagcagcagcagcagcagcagcagcag**caagag<br>actagccccaggcagcagcagcagcagcagggtgaggatggttctccccaagcccatc<br>gaagaggccccagaggctaggtggtgcaggatgaggaacagccaccttcacagc |

In Fig. 5, lane 28 shows the migration result of LH reaction product of sample M34; lane 27 shows the migration result of LH reaction product of sample M15; lane 26 shows the migration result of LH reaction product of sample M6; lane 25 shows the migration result of LH reaction product of sample U7; lane 24 shows the migration result of LH reaction product of sample M18; lane 23 shows the migration result of LH reaction product of sample M7; lane 22 shows the migration result of LH reaction product of sample M25; lane 21 shows the migration result of LH reaction product of sample M2.

As apparent from the result of Fig. 5, by the method using the conventional LH probe, signal bands of a plurality of non-specific reaction products were much detected. In addition, such tendency that band of the detected LH reaction product becomes non-uniformity was recognized, and identification of AR gene having different CAG-repeat is difficult, and accuracy of determination was significantly bad.

On the other hand, in case of using probe relevant to the present invention, as shown in Fig. 4, non-specific reaction product can hardly be found, and it was found that AR gene having different CAG-repeat can clearly be separated/detected.

## Claims

1. A method for detecting DNA having a microsatellite region, comprising:

   (1) contacting a probe, which does not have a nucleotide sequence complementary to said microsatellite region and hybridizes with nucleotide sequences on both sides of said microsatellite region and does not bind to said microsatellite region, with DNA having the microsatellite region, to form a hybrid of the above-mentioned DNA and the above-mentioned probe, which has a loop structure including said microsatellite region;
   (2) separating the obtained hybrid based on the difference of molecular weight, molecular structure and/or charge;
   (3) detecting said hybrid,

   wherein the number of repetitions of a unit as a standard of iteration in the microsatellite region is 5 to 100.

2. The method according to claim 1, wherein the probe is a sequence which is made by binding
   a complementary strand to a nucleotide sequence having 5 to 1000 nucleotides starting from a nucleotide distanced by 1 to 11 nucleotides from the 3'-terminal of the microsatellite region toward the 3'-terminal direction of the DNA having the microsatellite region, and
   a complementary strand to a nucleotide sequence having 5 to 1000 nucleotides starting from a nucleotide distanced by 1 to 11 nucleotides from the 5'-terminal of the microsatellite region toward the 5'-terminal direction of the DNA having the microsatellite region,
   so that the complementary nucleotide to the nucleotide distanced by 1 to 11 nucleotides the from 3'-terminal of the microsatellite region; and the complementary nucleotide to the nucleotide distanced by 1 to 11 nucleotides from the 5'-terminal of the microsatellite region, are bound.

3. The method according to claim 1 wherein the probe is a sequence which is made by binding
   a complementary strand to a nucleotide sequence having 5 to 1000 nucleotides starting from a nucleotide distanced by 1 nucleotide from the 3'-terminal of the microsatellite region toward the 3'-terminal direction of the DNA having the microsatellite region, and
a complementary strand to a nucleotide sequence having 5 to 1000 nucleotides starting from a nucleotide distanced by 1 nucleotide from the 5'-terminal of the microsatellite region toward the 5'-terminal direction of the DNA having the microsatellite region
so that the complementary nucleotide to the nucleotide distanced by 1 nucleotide from the 3'-terminal of the microsatellite region, and the complementary nucleotide to the nucleotide distanced by 1 nucleotide from the 5'-terminal of the microsatellite region are bound.

**4.** The method according to claim 1 wherein a method for separating the hybrid is an electrophoresis method.

## Patentansprüche

**1.** Verfahren zum Erfassen von DNS, die eine Mikrosatellitenregion aufweist, umfassend:

(1) Kontaktieren einer Sonde, die keine Nukleotidsequenz aufweist, die zu der Mikrosatellitenregion komplementär ist und mit Nukleotidsequenzen auf beiden Seiten der Mikrosatellitenregion hybridisiert und die keine Bindung mit der Mikrosatellitenregion eingeht, mit DNS, welche die Mikrosatellitenregion aufweist, um ein Hybrid aus der vorgenannten DNS und der vorgenannten Sonde zu bilden, der eine Schleifenstruktur aufweist, welche die Mikrosatellitenregion umfasst;
(2) Trennen des erhaltenen Hybrids auf Basis eines Unterschieds des Molekulargewichts, der Molekülstruktur und/oder der Ladung;
(3) Erfassen des Hybrids;

wobei die Anzahl an Wiederholungen einer Einheit als Standard der Iteration in der Mikrosatellitenregion bei 5 bis 100 liegt.

**2.** Verfahren nach Anspruch 1, wobei die Sonde eine Sequenz ist, hergestellt aus der Verbindung von
einem Strang, der komplementär ist zu einer Nukleotidsequenz mit 5 bis 1000 Nukleotiden, angefangen bei einem Nukleotid, das einen Abstand von 1 bis 11 Nukleotiden von dem 3'-Ende der Mikrosatellitenregion in Richtung des 3'-Endes der DNS hat, welche die Mikrosatellitenregion aufweist, und
einem Strang, der komplementär ist zu einer Nukleotidsequenz mit 5 bis 1000 Nukleotiden, angefangen bei einem Nukleotid, das einen Abstand von 1 bis 11 Nukleotiden vom 5'-Ende der Mikrosatellitenregion in Richtung 5'-Endes der DNS hat, welche die Mikrosatellitenregion aufweist,
so dass das Nukleotid, das zum Nukleotid komplementär ist, das vom 3'-Ende der Mikrosatellitenregion einen Abstand von 1 bis 11 Nukleotiden hat, und das Nukleotid, das zum Nukleotid komplementär ist, das vom 5'-Ende der Mikrosatellitenregion einen Abstand von 1 bis 11 Nukleotiden hat, verbunden sind.

**3.** Verfahren nach Anspruch 1, wobei die Sonde eine Sequenz ist, hergestellt aus der Verbindung von
einem Strang, der komplementär ist zu einer Nukleotidsequenz mit 5 bis 1000 Nukleotiden, angefangen bei einem Nukleotid, das einen Abstand von 1 Nukleotid vom 3'-Ende der Mikrosatellitenregion in Richtung des 3'-Endes der DNS hat, welche die Mikrosatellitenregion aufweist, und
einem Strang, der komplementär ist zu einer Nukleotidsequenz mit 5 bis 1000 Nukleotiden, angefangen bei einem Nukleotid, das einen Abstand von 1 Nukleotid vom 5'-Ende der Mikrosatellitenregion in Richtung des 5'-Endes der DNS hat, welche die Mikrosatellitenregion aufweist,
so dass das Nukleotid, das zum Nukleotid komplementär ist, das vom 3'-Ende der Mikrosatellitenregion einen Abstand von 1 Nukleotid hat, und das Nukleotid, das zum Nukleotid komplementär ist, das vom 5'-Ende der Mikrosatellitenregion einen Abstand von 1 Nukleotid hat, verbunden sind.

**4.** Verfahren nach Anspruch 1, wobei ein Verfahren zum Trennen des Hybrids ein Elektrophoreseverfahren ist.

## Revendications

**1.** Procédé de détection d'un ADN ayant une région microsatellite, comprenant :

(1) la mise en contact d'une sonde qui ne comporte pas de séquence nucléotidique complémentaire de ladite région microsatellite et s'hybride avec des séquences nucléotidiques des deux côtés de ladite région microsatellite et ne se lie pas à ladite région microsatellite, avec un ADN ayant la région microsatellite, pour former un hybride de l'ADN susmentionné et de la sonde susmentionnée, qui présente une structure de boucle comprenant ladite région microsatellite ;
(2) la séparation de l'hybride obtenu sur la base de la différence de masse moléculaire, de structure moléculaire et/ou de charge ;
(3) la détection dudit hybride,

dans lequel le nombre de répétitions d'une unité servant de norme d'itération dans la région microsatellite va de 5 à 100.

2. Procédé selon la revendication 1, dans lequel la sonde est une séquence qu'on fabrique en liant
un brin complémentaire d'une séquence nucléotidique comportant 5 à 1 000 nucléotides en partant d'un nucléotide situé à une distance de 1 à 11 nucléotides de la terminaison 3' de la région microsatellite et en allant vers la terminaison 3' de l'ADN ayant la région microsatellite, et
un brin complémentaire d'une séquence nucléotidique comportant 5 à 1 000 nucléotides en partant d'un nucléotide situé à une distance de 1 à 11 nucléotides de la terminaison 5' de la région microsatellite et en allant vers la terminaison 5' de l'ADN ayant la région microsatellite,
de sorte que le nucléotide complémentaire du nucléotide situé à une distance de 1 à 11 nucléotides de la terminaison 3' de la région microsatellite ; et le nucléotide complémentaire du nucléotide situé à une distance de 1 à 11 nucléotides de la terminaison 5' de la région microsatellite, sont liés.

3. Procédé selon la revendication 1, dans lequel la sonde est une séquence qu'on fabrique en liant
un brin complémentaire d'une séquence nucléotidique comportant 5 à 1 000 nucléotides en partant d'un nucléotide situé à une distance de 1 nucléotide de la terminaison 3' de la région microsatellite et en allant vers la terminaison 3' de l'ADN ayant la région microsatellite, et
un brin complémentaire d'une séquence nucléotidique comportant 5 à 1 000 nucléotides en partant d'un nucléotide situé à une distance de 1 nucléotide de la terminaison 5' de la région microsatellite et en allant vers la terminaison 5' de l'ADN ayant la région microsatellite,
de sorte que le nucléotide complémentaire du nucléotide situé à une distance de 1 nucléotide de la terminaison 3' de la région microsatellite, et le nucléotide complémentaire du nucléotide situé à une distance de 1 nucléotide de la terminaison 5' de la région microsatellite, sont liés.

4. Procédé selon la revendication 1 dans lequel le procédé de séparation de l'hybride est un procédé d'électrophorèse.

Figure 1

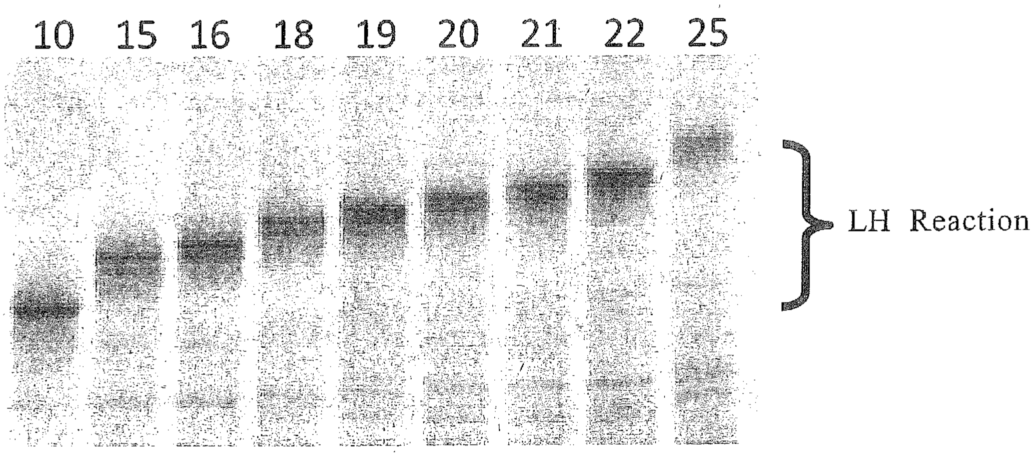


Figure 2

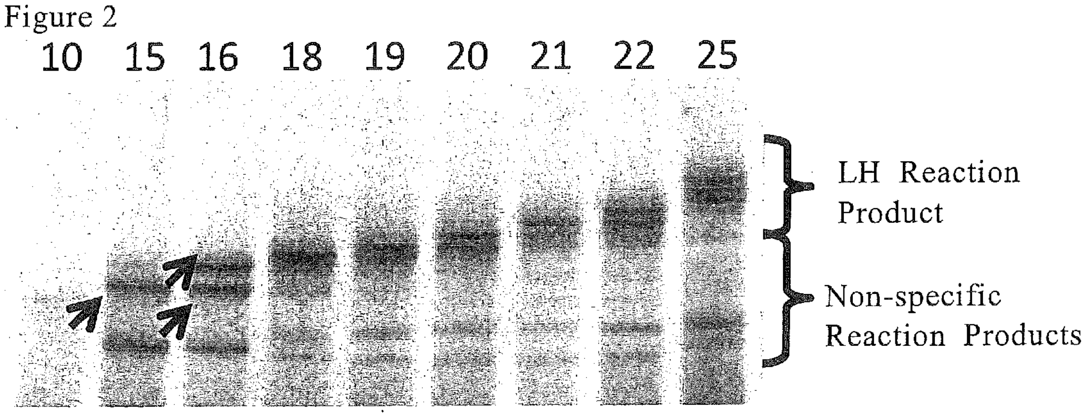


Figure 3

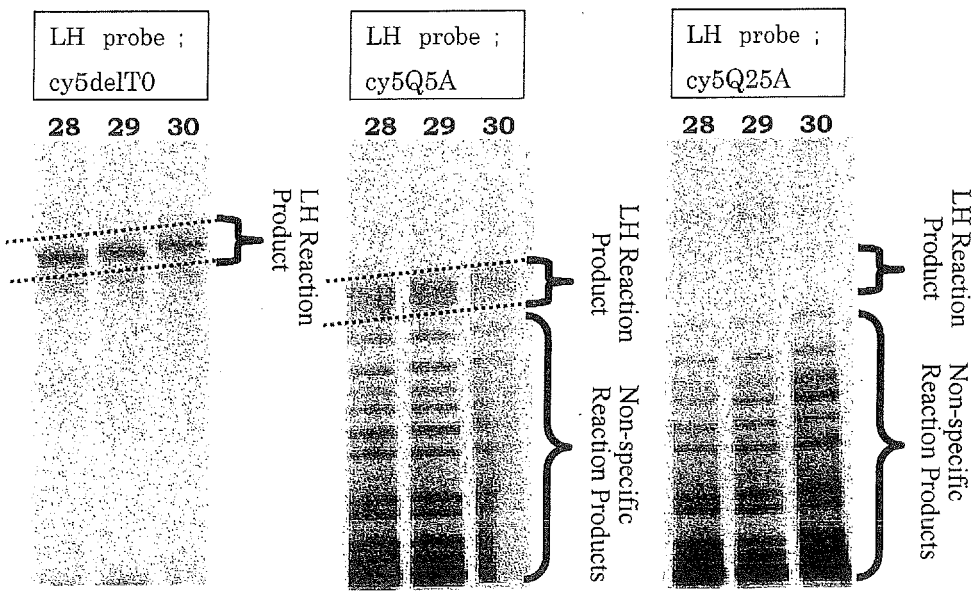

Figure 4

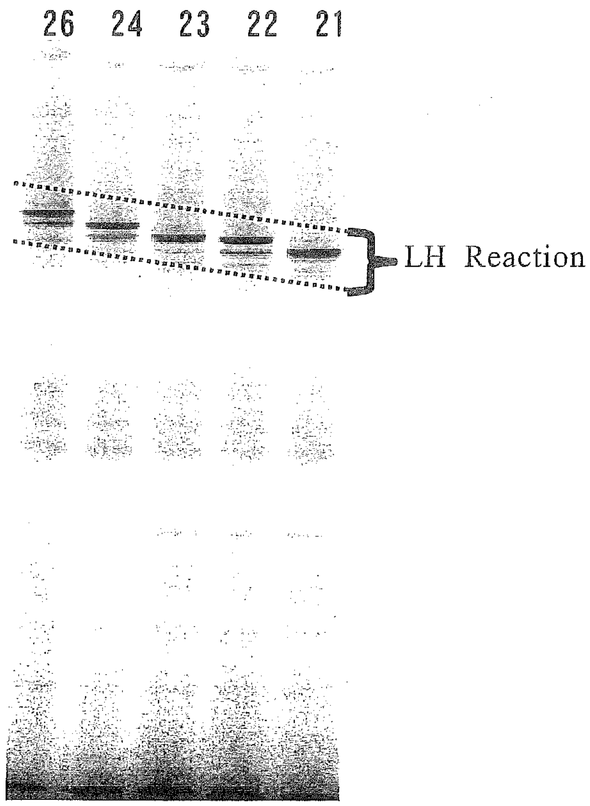


Figure 5

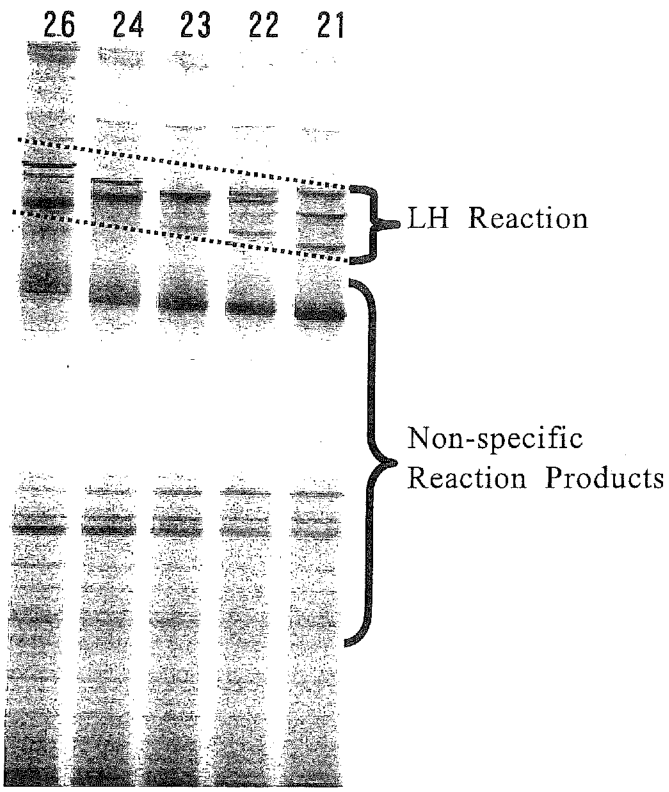

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007061080 A
- WO 03014398 A
- WO 03031580 A
- US 5585236 A
- US 5772889 A
- US 5972222 A
- JP 2009125009 A
- WO 2007027495 A
- US 4981977 A
- US 5268486 A
- US 5486616 A
- US 6083485 A
- WO 2006047452 A
- WO 13764078 A
- JP 2012065232 B


### Non-patent literature cited in the description


- *Clinica Chimica Acta,* vol. 412, 1688-1672
- **BOOM et al.** *J. Clin. Microbiol.,* 1990, vol. 28, 495-503
- *Proc. Natl. Acad. Sci. USA,* 1979, vol. 76-2, 615-619
- *Nucleic Acids Research,* 1991, vol. 19, 3749
- *Bio Techniques,* 1994, vol. 16, 1134-1137
- *Anal. Chem.,* 1993, vol. 65 (5), 613-616
- *Panasonic Technical Journal,* October 2011, vol. 57 (3

SEQUENCE LISTING

<110> WAKO PURE CHEMICAL INDUSTRIES, LTD.

<120> Method for detecting DNA having microsatellite region

<130> WP0141

<140> 13764078.5
<141> 2013-03-22

<150> JP2012065232
<151> 2012-03-22

<160> 43

<170> PatentIn version 3.3

<210> 1
<211> 75
<212> DNA
<213> Artificial

<220>
<223> TOMM40 gene

<400> 1

**taggcattcg aagccagccc gggcaacatg gtgagacccc atctcaaaaa aaaaagccag 60**

**atgcaatggc tcatg 75**

<210> 2
<211> 80
<212> DNA
<213> Artificial

<220>
<223> TOMM40 gene

<400> 2

**taggcattcg aagccagccc gggcaacatg gtgagacccc atctcaaaaa aaaaaaaaaa 60**

**gccagatgca atggctcatg 80**

<210> 3
<211> 81
<212> DNA
<213> Artificial

<220>
<223> TOMM40 gene

<400> 3

**taggcattcg aagccagccc gggcaacatg gtgagacccc atctcaaaaa aaaaaaaaaa 60**

**agccagatgc aatggctcat g 81**

<210> 4
<211> 83
<212> DNA
<213> Artificial

<220>
<223> TOMM40 gene

<400> 4

**taggcattcg aagccagccc gggcaacatg gtgagacccc atctcaaaaa aaaaaaaaaa 60**

**aaagccagat gcaatggctc atg 83**

<210> 5
<211> 84
<212> DNA
<213> Artificial

<220>
<223> TOMM40 gene

<400> 5

**taggcattcg aagccagccc gggcaacatg gtgagacccc atctcaaaaa aaaaaaaaaa 60**

**aaaagccaga tgcaatggct catg 84**

<210> 6
<211> 85
<212> DNA
<213> Artificial

<220>
<223> TOMM40 gene

<400> 6

taggcattcg aagccagccc gggcaacatg gtgagacccc atctcaaaaa aaaaaaaaaa 60

aaaaagccag atgcaatggc tcatg 85

<210> 7
<211> 86
<212> DNA
<213> Artificial

<220>
<223> TOMM40 gene

<400> 7

taggcattcg aagccagccc gggcaacatg gtgagacccc atctcaaaaa aaaaaaaaaa 60

aaaaaagcca gatgcaatgg ctcatg 86

<210> 8
<211> 87
<212> DNA
<213> Artificial

<220>
<223> TOMM40 gene

<400> 8

taggcattcg aagccagccc gggcaacatg gtgagacccc atctcaaaaa aaaaaaaaaa 60

aaaaaaagcc agatgcaatg gctcatg 87

<210> 9
<211> 90
<212> DNA
<213> Artificial

<220>
<223> TOMM40 gene

<400> 9

taggcattcg aagccagccc gggcaacatg gtgagacccc atctcaaaaa aaaaaaaaaa 60

aaaaaaaaaa gccagatgca atggctcatg 90

<210> 10
<211> 93
<212> DNA
<213> Artificial

<220>
<223> TOMM40 gene

<400> 10

taggcattcg aagccagccc gggcaacatg gtgagacccc atctcaaaaa aaaaaaaaaa 60

aaaaaaaaaa aaagccagat gcaatggctc atg 93

<210> 11
<211> 94
<212> DNA
<213> Artificial

<220>
<223> TOMM40 gene

<400> 11

taggcattcg aagccagccc gggcaacatg gtgagacccc atctcaaaaa aaaaaaaaaa 60

aaaaaaaaaa aaaagccaga tgcaatggct catg 94

<210> 12
<211> 95
<212> DNA
<213> Artificial

<220>
<223> TOMM40 gene

<400> 12

taggcattcg aagccagccc gggcaacatg gtgagacccc atctcaaaaa aaaaaaaaaa 60

aaaaaaaaaa aaaaagccag atgcaatggc tcatg 95

<210> 13
<211> 71
<212> DNA
<213> Artificial

<220>
<223> TOMM40 gene

<400> 13

gacctcaagc tgtcctcttg ccccagccct ccaaagcatt gggattactg gcatgagcca 60

ttgcatctgg c 71

<210> 14
<211> 22
<212> DNA
<213> Artificial

<220>

<223> PCR primer

<400> 14
gacctcaagc tgtcctcttg cc 22

<210> 15
<211> 21
<212> DNA
<213> Artificial

<220>
<223> PCR primer

<400> 15
taggcattcg aagccagccc g 21

<210> 16
<211> 126
<212> DNA
<213> Artificial

<220>
<223> TOMM40 gene

<400> 16

**taggcattcg aagccagccc gggcaacatg gtgagacccc atctcaaaaa aaaaagccag 60**

**atgcaatggc tcatgccagt aatcccaatg ctttggaggg ctggggcaag aggacagctt 120**

**gaggtc 126**

<210> 17
<211> 131
<212> DNA
<213> Artificial

<220>
<223> TOMM40 gene

<400> 17

**taggcattcg aagccagccc gggcaacatg gtgagacccc atctcaaaaa aaaaaaaaaa 60**

**gccagatgca atggctcatg ccagtaatcc caatgctttg gagggctggg gcaagaggac 120**

**agcttgaggt c 131**

<210> 18
<211> 132
<212> DNA
<213> Artificial

<220>
<223> TOMM40 gene

<400> 18

```
taggcattcg aagccagccc gggcaacatg gtgagacccc atctcaaaaa aaaaaaaaaa      60
agccagatgc aatggctcat gccagtaatc ccaatgcttt ggagggctgg ggcaagagga     120
cagcttgagg tc                                                         132
```

<210> 19
<211> 134
<212> DNA
<213> Artificial

<220>
<223> TOMM40 gene

<400> 19

```
taggcattcg aagccagccc gggcaacatg gtgagacccc atctcaaaaa aaaaaaaaaa      60
aaagccagat gcaatggctc atgccagtaa tcccaatgct ttggagggct ggggcaagag     120
gacagcttga ggtc                                                       134
```

<210> 20
<211> 135
<212> DNA
<213> Artificial

<220>
<223> TOMM40 gene

<400> 20

```
taggcattcg aagccagccc gggcaacatg gtgagacccc atctcaaaaa aaaaaaaaaa      60
aaaagccaga tgcaatggct catgccagta atcccaatgc tttggagggc tggggcaaga     120
ggacagcttg aggtc                                                      135
```

<210> 21
<211> 136
<212> DNA
<213> Artificial

<220>
<223> TOMM40 gene

<400> 21

```
taggcattcg aagccagccc gggcaacatg gtgagacccc atctcaaaaa aaaaaaaaaa      60
aaaaagccag atgcaatggc tcatgccagt aatcccaatg ctttggaggg ctggggcaag     120
aggacagctt gaggtc                                                     136
```

<210> 22
<211> 137

<212> DNA
<213> Artificial

<220>
<223> TOMM40 gene

<400> 22

```
taggcattcg aagccagccc gggcaacatg gtgagacccc atctcaaaaa aaaaaaaaaa     60
aaaaaagcca gatgcaatgg ctcatgccag taatcccaat gctttggagg gctggggcaa    120
gaggacagct tgaggtc                                                   137
```

<210> 23
<211> 138
<212> DNA
<213> Artificial

<220>
<223> TOMM40 gene

<400> 23

```
taggcattcg aagccagccc gggcaacatg gtgagacccc atctcaaaaa aaaaaaaaaa     60
aaaaaaagcc agatgcaatg gctcatgcca gtaatcccaa tgctttggag ggctggggca    120
agaggacagc ttgaggtc                                                  138
```

<210> 24
<211> 141
<212> DNA
<213> Artificial

<220>
<223> TOMM40 gene

<400> 24

```
taggcattcg aagccagccc gggcaacatg gtgagacccc atctcaaaaa aaaaaaaaaa     60
aaaaaaaaaa gccagatgca atggctcatg ccagtaatcc caatgctttg gagggctggg    120
gcaagaggac agcttgaggt c                                              141
```

<210> 25
<211> 144
<212> DNA
<213> Artificial

<220>
<223> TOMM40 gene

<400> 25

taggcattcg aagccagccc gggcaacatg gtgagacccc atctcaaaaa aaaaaaaaaa 60

aaaaaaaaaa aaagccagat gcaatggctc atgccagtaa tcccaatgct ttggagggct 120

ggggcaagag gacagcttga ggtc 144

<210> 26
<211> 145
<212> DNA
<213> Artificial

<220>
<223> TOMM40 gene

<400> 26

taggcattcg aagccagccc gggcaacatg gtgagacccc atctcaaaaa aaaaaaaaaa 60

aaaaaaaaaa aaaagccaga tgcaatggct catgccagta atcccaatgc tttggagggc 120

tggggcaaga ggacagcttg aggtc 145

<210> 27
<211> 146
<212> DNA
<213> Artificial

<220>
<223> TOMM40 gene

<400> 27

taggcattcg aagccagccc gggcaacatg gtgagacccc atctcaaaaa aaaaaaaaaa 60

aaaaaaaaaa aaaaagccag atgcaatggc tcatgccagt aatcccaatg ctttggaggg 120

ctggggcaag aggacagctt gaggtc 146

<210> 28
<211> 90
<212> DNA
<213> Artificial

<220>
<223> Probe

<400> 28

gacctcaagc tgtcctcttg ccccagccct ccaaagcatt gggattactg gcatgagcca 60

ttgcatctgg cgagatgggg tctcaccatg 90

<210> 29
<211> 75
<212> DNA
<213> Artificial

<220>
<223> Probe

<400> 29

**taggcattcg aagccagccc gggcaacatg gtgagacccc atctcaaaaa aaaaagccag 60**

**atgcaatggc tcatg 75**

<210> 30
<211> 70
<212> DNA
<213> Artificial

<220>
<223> Probe

<400> 30

**taggcattcg aagccagccc gggcaacatg gtgagacccc atctcaaaaa gccagatgca 60**

**atggctcatg 70**

<210> 31
<211> 90
<212> DNA
<213> Artificial

<220>
<223> Probe

<400> 31

**taggcattcg aagccagccc gggcaacatg gtgagacccc atctcaaaaa aaaaaaaaaa 60**

**aaaaaaaaaa gccagatgca atggctcatg 90**

<210> 32
<211> 20
<212> DNA
<213> Artificial

<220>
<223> Primer

<400> 32
agcgtgcgcg aagtgatcca 20

<210> 33
<211> 20
<212> DNA
<213> Artificial

<220>
<223> Primer

<400> 33
atgggcttgg ggagaaccat 20

<210> 34
<211> 283
<212> DNA
<213> Artificial

<220>
<223> Androgen receptor gene

<400> 34

agcgtgcgcg aagtgatcca gaacccgggc cccaggcacc cagaggccgc gagcgcagca 60

cctcccggcg ccagtttgct gctgctgcag cagcagcagc agcagcagca gcagcagcag 120

cagcagcagc agcagcagca gcagcagcag cagcagcagc agcagcaaga gactagcccc 180

aggcagcagc agcagcagca gggtgaggat ggttctcccc aagcccatcg aagaggcccc 240

agaggctagg tggtgcagga tgaggaacag ccaccttcac agc 283

<210> 35
<211> 277
<212> DNA
<213> Artificial

<220>
<223> Androgen receptor gene

<400> 35

agcgtgcgcg aagtgatcca gaacccgggc cccaggcacc cagaggccgc gagcgcagca 60

cctcccggcg ccagtttgct gctgctgcag cagcagcagc agcagcagca gcagcagcag 120

cagcagcagc agcagcagca gcagcagcag cagcagcagc aagagactag ccccaggcag 180

cagcagcagc agcagggtga ggatggttct ccccaagccc atcgaagagg ccccagaggc 240

taggtggtgc aggatgagga acagccacct tcacagc 277

<210> 36
<211> 274
<212> DNA
<213> Artificial

<220>
<223> Androgen receptor gene

<400> 36

```
agcgtgcgcg aagtgatcca gaacccgggc cccaggcacc cagaggccgc gagcgcagca     60
cctcccggcg ccagtttgct gctgctgcag cagcagcagc agcagcagca gcagcagcag    120
cagcagcagc agcagcagca gcagcagcag cagcagcaag agactagccc caggcagcag    180
cagcagcagc agggtgagga tggttctccc caagcccatc gtagaggccc cacaggctac    240
ctggtcctgg atgaggaaca gcaaccttca cagc                                274
```

<210> 37
<211> 271
<212> DNA
<213> Artificial

<220>
<223> Androgen receptor gene

<400> 37

```
agcgtgcgcg aagtgatcca gaacccgggc cccaggcacc cagaggccgc gagcgcagca     60
cctcccggcg ccagtttgct gctgctgcag cagcagcagc agcagcagca gcagcagcag    120
cagcagcagc agcagcagca gcagcagcag cagcaagaga ctagccccag gcagcagcag    180
cagcagcagg gtgaggatgg ttctccccaa gcccatcgta gaggccccac aggctacctg    240
gtcctggatg aggaacagca accttcacag c                                   271
```

<210> 38
<211> 268
<212> DNA
<213> Artificial

<220>
<223> Androgen receptor gene

<400> 38

```
agcgtgcgcg aagtgatcca raacccgggc cccaggcacc cagaggccgc gagcgcagca     60
cctcccggcg ccagtttgct gctgctgcag cagcagcagc agcagcagca gcagcagcag    120
cagcagcagc agcagcagca gcagcagcag caagagacta gccccaggca gcagcagcag    180
cagcagggtg aggatggttc tccccaagcc catcgtagag gccccacagg ctacctggtc    240
ctggatgagg aacagcaacc ttcacagc                                       268
```

<210> 39
<211> 64
<212> DNA
<213> Artificial

<220>
<223> Probe

<400> 39

atgggcttgg ggagaaccat cctcaccctt gcctggggct agtctctgca gcagcaaact 60

ggcg 64

<210> 40
<211> 104
<212> DNA
<213> Artificial

<220>
<223> Probe

<400> 40

atgggcttgg ggagaaccat cctcaccctt gcctggggct agtctcttgc tgctgctgct 60

gctgctgctg ctgctgctgc tgctgcagca gcagcaaact ggcg 104

<210> 41
<211> 289
<212> DNA
<213> Artificial

<220>
<223> Androgen receptor gene

<400> 41

agcgtgcgcg aagtgatcca gaacccgggc cccaggcacc cagaggccgc gagcgcagca 60

cctcccggcg ccagtttgct gctgctgcag cagcagcagc agcagcagca gcagcagcag 120

cagcagcagc agcagcagca gcagcagcag cagcagcagc agcagcagca gcaagagact 180

agccccaggc agcagcagca gcagcagggt gaggatggtt ctccccaagc ccatcgaaga 240

ggccccagag gctaggtggt gcaggatgag gaacagccac cttcacagc 289

<210> 42
<211> 286
<212> DNA
<213> Artificial

<220>
<223> Androgen receptor gene

<400> 42

```
agcgtgcgcg aagtgatcca gaacccgggc cccaggcacc cagaggccgc gagcgcagca    60
cctcccggcg ccagtttgct gctgctgcag cagcagcagc agcagcagca gcagcagcag   120
cagcagcagc agcagcagca gcagcagcag cagcagcagc agcagcagca agagactagc   180
cccaggcagc agcagcagca gcagggtgag gatggttctc cccaagccca tcgaagaggc   240
cccagaggct aggtggtgca ggatgaggaa cagccacctt cacagc                  286
```

<210> 43
<211> 280
<212> DNA
<213> Artificial

<220>
<223> Androgen receptor gene

<400> 43

```
agcgtgcgcg aagtgatcca gaacccgggc cccaggcacc cagaggccgc gagcgcagca    60
cctcccggcg ccagtttgct gctgctgcag cagcagcagc agcagcagca gcagcagcag   120
cagcagcagc agcagcagca gcagcagcag cagcagcagc agcaagagac tagccccagg   180
cagcagcagc agcagcaggg tgaggatggt tctccccaag cccatcgaag aggccccaga   240
ggctaggtgg tgcaggatga ggaacagcca ccttcacagc                         280
```